# EUROPÄISCHE PATENTSCHRIFT

(11) **EP 3 230 427 B1**
(45) Veröffentlichungstag und Bekanntmachung des Hinweises auf die Patenterteilung: **06.03.2024**
(21) Anmeldenummer: 15790155.4
(22) Anmeldetag: 05.11.2015
(51) Int. Cl.: C12N 9/00, C12N 15/00, C11D 3/386, C12N 9/28

(54) **WASCH- ODER REINIGUNGSMITTEL MIT SPEZIELLER ALPHA-AMYLASE UND DEFINIERTER WASSERAKTIVITÄT**
LAUNDRY AND CLEANING COMPOSITIONS WITH SPECIAL ALPHA-AMYLASE AND DEFINED WATER ACTIVITY
COMPOSITIONS DE LAVAGE ET NETTOYAGE AVEC ALPHA-AMYLASE SPÉCIALE ET ACTIVITÉ DE L'EAU DÉFINÉ

(30) Priorität: 10.12.2014 DE 102014225478
(43) Veröffentlichungstag der Anmeldung: 18.10.2017
(73) Patentinhaber: Henkel AG & Co. KGaA, 40589 Düsseldorf (DE)
(72) Erfinder: ANTIR, Marouane, 40591 Düsseldorf (DE); SCHÜMANN, Sabine, 41470 Neuss (DE); BASTIGKEIT, Thorsten, 42279 Wuppertal (DE); SEILER, Martina, 47228 Duisburg (DE); O'CONNELL, Timothy, 40547 Düsseldorf (DE); MEIER, Frank, 40589 Düsseldorf (DE); WEINRICH, Dirk, 41517 Grevenbroich (DE); KÖLLING, Leyla, 42659 Solingen (DE); BELLOMI, Luca, 40627 Düsseldorf (DE); EDWARDS, Sheila, 40599 Düsseldorf (DE)
(86) Internationale Anmeldenummer: PCT/EP2015/075798
(87) Internationale Veröffentlichungsnummer: WO 2016/091494

(56) Entgegenhaltungen:
- WO-A1-2013/171095
- WO-A1-2014/162001
- WO-A1-2014/183920
- WO-A2-2013/063460
- A. Crutzen, M.L. Douglass: ; "A" In: U.Zoller: "Handbook of Detergents", 14. April 1999 (1999-04-14), Marcel Dekker, Inc., New York, XP002754021, ISBN: 0-8247-1417-2 Seite 674, Seite 674
- CRUTZEN A ET AL: "Handbook of Detergents, Part A: Properties , C. Stabilization Systems", 14 April 1999 (1999-04-14), HANDBOOK OF DETERGENTS; [SURFACTANT SCIENCE SERIES , ISSN 0081-9603], MARCEL DEKKER, INC, NEW YORK [U.A.], PAGE(S) 674, XP002754021, ISBN: 978-0-8247-1417-8

## Beschreibung

Die vorliegende Erfindung betrifft die Reinigung von Oberflächen, insbesondere von Textilien, sowie die Bereitstellung flüssiger Zusammensetzungen für diesen Zweck.

Meistens wird für die Entfernung verschiedenartigster Anschmutzungen auf Substraten ein und dasselbe Wasch- und Reinigungsmittel angewendet. Um den Anforderungen einer größtmöglichen effektiven Fleckentfernung zu genügen, umfassen Wasch- und Reinigungsmittel verschiedenartige aktive Inhaltsstoffe. Der Fachmann kennt beispielsweise Anschmutzungen, die sich durch den Einsatz von Enzymen verbessert entfernen lassen, die sogenannten enzymsensitiven Anschmutzungen. Für die Entfernung enzymsensitiver Anschmutzungen werden Wasch- oder Reinigungsmitteln daher neben den zur Schmutzentfernung üblich genutzten Tensiden zusätzlich beispielsweise die Enzyme Protease, Lipase, Amylase, Mannanase zugesetzt.

Zur Entfernung von stärkebasierten Anschmutzungen enthalten Wasch- oder Reinigungsmittel oft das Enzym α-Amylase. Für Amylasen können synonyme Begriffe verwendet werden, beispielsweise 1,4-alpha-D-Glucan-Glucanohydrolase oder Glycogenase. Bevorzugte Amylasen sind oftmals α-Amylasen. Entscheidend dafür, ob ein Enzym eine α-Amylase im Sinne der Erfindung ist, ist deren Fähigkeit zur Hydrolyse von α(1-4)-Glykosidbindungen in der Amylose der Stärke.

Im Stand der Technik bekannte Amylasen sowie insbesondere auch deren für den Einsatz in Wasch- oder Reinigungsmitteln verbesserte Weiterentwicklungen sind im Folgenden geschildert. Die α-Amylase aus Bacillus licheniformis (erhältlich als Termamyl^{®} von Novozymes oder Purastar^{®}ST von dem Unternehmen Danisco/Genencor) und deren Weiterentwicklungsprodukte Duramyl^{®} und Termamyl^{®}ultra (Novozymes), Purastar^{®}OxAm (Danisco/Genencor) und Keistase^{®} (Daiwa Seiko Inc.), sowie die α-Amylasen aus Bacillus amyloliquefaciens oder aus Bacillus stearothermophilus sind geeignete Amylasen. Die α-Amylase von Bacillus amyloliquefaciens wird von dem Unternehmen Novozymes unter dem Namen BAN^{®} vertrieben, und abgeleitete Varianten von der α-Amylase aus Bacillus stearothermophilus unter den Namen BSG^{®} und Novamyl^{®}, ebenfalls von dem Unternehmen Novozymes. Weitere bekannte Amylasen sind die α-Amylase aus Bacillus sp. A 7-7 (DSM 12368) und die Cyclodextrin-Glucanotransferase (CGTase) aus Bacillus agaradherens (DSM 9948) sowie die unter den Handelsnamen Fungamyl^{®} von dem Unternehmen Novozymes erhältlichen Weiterentwicklungen der α-Amylase aus Aspergillus niger und A. oryzae. Weitere vorteilhaft in Wasch- und Reinigungsmitteln einsetzbare Handelsprodukte sind beispielsweise die Amylase-LT^{®} und Stainzyme^{®} oder Stainzyme ultra^{®} bzw. Stainzyme plus^{®}, letztere ebenfalls von dem Unternehmen Novozymes. Auch durch Punktmutationen erhältliche Varianten dieser Enzyme können in Wasch- und Reinigungsmitteln eingesetzt werden. Besonders prominente Amylasen sind offenbart in den internationalen Offenlegungsschriften WO 00/60060, WO 03/002711, WO 03/054177 und WO07/079938.

Die Aktivität der Amylase während des Wasch- bzw. Reinigungsvorganges hängt von unterschiedlichen Faktoren ab. Neben der Temperatur und dem pH-Wert des Wasch- bzw. Reinigungsmediums ist die Lagerstabilität des Enzyms im Wasch- oder Reinigungsmittel ein entscheidender Faktor. Die Enzymaktivität soll stets den Zeitraum von der Produktion des Wasch- oder Reinigungsmittels bis hin zur letzten Nutzung durch den Verbraucher in hohem Masse gewährleistet sein.

Die Anmelderin hat es sich daher zur Aufgabe gemacht, flüssige Zusammensetzungen (insbesondere Wasch- oder Reinigungszusammensetzungen für Textilien) bereitzustellen, die eine hervorragende Waschkraft mit Blick auf die Entfernung stärkebasierter Anschmutzungen aufweisen. Diese Effekte sollen bei möglichst geringen Anwendungstemperaturen, insbesondere zwischen 10°C und 40°C, erzielt werden. Ferner soll die durch das Enzym bewirkte Waschkraft möglichst vollständig über den Lagerzeitraum des Wasch- oder Reinigungsmittels erhalten bleiben.

WO 2014/183920 A1 offenbart ebenso wie WO 2013/063460 A2 flüssige Zusammensetzungen mit Amylasen bestimmter Sequenzen WO 2014/62001 A1 lehrt amylasehaltige Zusammensetzungen mit niedrigem Wassergehalt, und WO 2013/171095 A1 lehrt ebenfalls amylasehaltige Zusammensetzungen und gibt deren Wasseraktivität an. A. Crutzen und M.L. Douglass offenbaren einen Zusammenhang zwischen Wasseraktivität und Enzymstabilität in flüssigen Zusammensetzungen (U. Zoller, "Handbook of Detergents", 14. April 1999, Marcel Dekker, Inc., New York, Seite 674).

Ein erster Gegenstand der Erfindung ist eine flüssige Zusammensetzung, insbesondere für die Wäsche oder Reinigung von Textilien, enthaltend
(a) mindestens ein Tensid, und
(b) mindestens eine α-Amylase, die zu der in SEQ ID NO.1 angegeben Sequenz über deren Gesamtlänge zu mindestens 89% und zunehmend bevorzugt zu mindestens 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99%, 99,5% und bis zu 100% identisch ist und in der Zählung gemäß SEQ ID NO. 1 an einer oder mehreren der Positionen 180, 181, 182, 183 und 184 Deletionen aufweist,
mit der Maßgabe, dass die Zusammensetzung bei 25°C und 1013 mbar eine Wasseraktivität a_{w} von mehr als 0,3 bis maximal 0,9 aufweist, dadurch gekennzeichnet, dass der Gehalt an Wasser weniger als 30 Gew.-% beträgt.

Im Sinne der Erfindung gilt für die Definition eines Zahlenbereichs, welcher "zwischen" zwei Bereichsgrenzen liegen soll, dem allgemeinen Sprachgebrauch folgend, dass die die Bereichsgrenzen nicht mit eingeschlossen sind. Zahlenbereiche, die von einer Bereichsgrenze bis zu einer anderen Bereichsgrenze definiert sind, schließen die Bereichsgrenzen mit ein.

Die erfindungsgemäße Zusammensetzung ist bei 25°C und 1013 mbar flüssig.

Zur Entfaltung einer Reinigungs- oder Waschleistung enthalten die erfindungsgemäßen Zusammensetzungen mindestens ein Tensid, besonders bevorzugt eine Mischung mehrerer Tenside aus unterschiedlichen Stoffklassen.

Dabei ist es für die Erfindung bevorzugt, wenn die Gesamtmenge an Tensid 2,0 bis 70 Gew.-%, bevorzugt 5,0 bis 60,0 Gew.-%, weiter bevorzugt von 10,0 bis 40,0 Gew.-%, besonders bevorzugt von 15,0 bis 36,0 Gew.-%, jeweils bezogen auf das Gesamtgewicht der Zusammensetzung, beträgt.

Als Tensid wird erfindungsgemäß bevorzugt mindestens ein anionisches Tensid eingesetzt.

Als anionische Tenside eignen sich in den Zusammensetzungen alle anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Geeignete anionische Tenside liegen vorzugsweise in Form der Natrium-, Kalium- und Ammoniumsowie der Mono-, Di- und Trialkanolammoniumsalze mit 2 bis 4 C-Atomen in der Alkanolgruppe vor.

Bevorzugte Zusammensetzungen enthalten auf das Gesamtgewicht der Zusammensetzung bezogen anionisches Tensid in einer Gesamtmenge von 1,0 bis 35,0 Gew.-%, bevorzugt von 5,0 bis 30,0 Gew.-%, besonders bevorzugt von 10,0 bis 25,0 Gew.-%.

Bevorzugte anionische Tenside in den Zusammensetzungen sind Alkylsulfate, Alkylpolyglykolethersulfate und Ethercarbonsäuren, jeweils mit 10 bis 18 C-Atomen in der Alkylgruppe und bis zu 12 Glykolethergruppen im Molekül.

Bevorzugte erfindungsgemäß eingesetzte Zusammensetzungen enthalten mindestens ein Tensid der Formel

R¹-O-(AO)ₙ-SO₃⁻X⁺.

In dieser Formel steht R¹ für einen linearen oder verzweigten, substituierten oder unsubstituierten Alkyl-, Aryl- oder Alkylarylrest, vorzugsweise für einen linearen, unsubstituierten Alkylrest, besonders bevorzugt für einen Fettalkoholrest. Bevorzugte Reste R¹ sind ausgewählt aus Decyl-, Undecyl-, Dodecyl-, Tridecyl-, Tetradecyl, Pentadecyl-, Hexadecyl-, Heptadecyl-, Octadecyl-, Nonadecyl-, Eicosylresten und deren Mischungen, wobei die Vertreter mit gerader Anzahl an C-Atomen bevorzugt sind. Besonders bevorzugte Reste R¹ sind abgeleitet von C₁₂-C₁₈-Fettalkoholen, beispielsweise von Kokosfettalkohol, Talgfettalkohol, Lauryl-, Myristyl-, Cetyl- oder Stearylalkohol oder von C₁₀-C₂₀-Oxoalkoholen.

AO steht für eine Ethylenoxid- (EO) oder Propylenoxid- (PO) Gruppierung, vorzugsweise für eine Ethylenoxidgruppierung. Der Index n steht für eine ganze Zahl von 1 bis 50, vorzugsweise von 1 bis 20 und insbesondere von 2 bis 10. Ganz besonders bevorzugt steht n für die Zahlen 2, 3, 4, 5, 6, 7 oder 8. X steht für ein einwertiges Kation oder den n-ten Teil eines n-wertigen Kations, bevorzugt sind dabei die Alkalimetallionen und darunter Na⁺ oder K⁺, wobei Na⁺ äußerst bevorzugt ist. Weitere Kationen X+ können ausgewählt sein aus NH₄⁺, ½ Zn²⁺,½ Mg²⁺,½ Ca²⁺,½ Mn²⁺, und deren Mischungen.

Zusammenfassend enthalten besonders bevorzugte Zusammensetzungen mindestens ein anionisches Tensid, ausgewählt aus Fettalkoholethersulfaten der Formel A-1 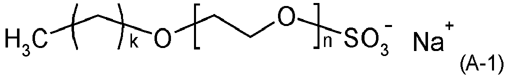 mit k = 11 bis 19, n = 2, 3, 4, 5, 6, 7 oder 8. Ganz besonders bevorzugte Vertreter sind Na-C₁₂₋₁₄ Fettalkoholethersulfate mit 2 EO (k = 11-13, n = 2 in Formel A-1).

Bevorzugte Zusammensetzungen enthalten bezogen auf die Gesamtmenge der Zusammensetzung 1,0 bis 15 Gew.-%, vorzugsweise 2,5 bis 12,5 Gew.-%, weiter bevorzugt 5,0 bis 10,0 Gew.-% Fettalkoholethersulfat(e) (jeweils insbesondere der Formel A1).

Weitere bevorzugte Zusammensetzungen enthalten zusätzlich oder alternativ (insbesondere zusätzlich) mindestens ein Tensid der Formel (A-2)

R³-A-SO₃⁻Y⁺ (A-2).

In dieser Formel steht R³ für einen linearen oder verzweigten, substituierten oder unsubstituierten Alkyl-, Aryl- oder Alkylarylrest und die Gruppierung -A- für -O- oder eine chemische Bindung. In anderen Worten lassen sich durch die vorstehende Formel Sulfat- (A = O) oder Sulfonat- (A = chemische Bindung) -tenside beschreiben. In Abhängigkeit von der Wahl der Gruppierung A sind bestimmte Reste R³ bevorzugt. Bei den Sulfattensiden (A = O) steht R³ vorzugsweise für einen linearen, unsubstituierten Alkylrest, besonders bevorzugt für einen Fettalkoholrest. Bevorzugte Reste R¹ sind ausgewählt aus Decyl-, Undecyl-, Dodecyl-, Tridecyl-, Tetradecyl, Pentadecyl-, Hexadecyl-, Heptadecyl-, Octadecyl-, Nonadecyl-, Eicosylresten und deren Mischungen, wobei die Vertreter mit gerader Anzahl an C-Atomen bevorzugt sind. Besonders bevorzugte Reste R¹ sind abgeleitet von C₁₂-C₁₈-Fettalkoholen, beispielsweise von Kokosfettalkohol, Talgfettalkohol, Lauryl-, Myristyl-, Cetyl- oder Stearylalkohol oder von C₁₀-C₂₀-Oxoalkoholen. Y steht für ein einwertiges Kation oder den n-ten Teil eines n-wertigen Kations, bevorzugt sind dabei die Alkalimetallionen und darunter Na⁺ oder K⁺, wobei Na⁺ äußerst bevorzugt ist. Weitere Kationen Y+ können ausgewählt sein aus NH₄⁺, ½ Zn²⁺,½ Mg²⁺,½ Ca²⁺,½ Mn²⁺, und deren Mischungen.

Solche besonders bevorzugten Tenside sind ausgewählt aus Fettalkoholsulfaten der Formel A-2a 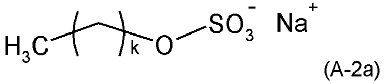 mit k = 11 bis 19. Ganz besonders bevorzugte Vertreter sind Na-C₁₂₋₁₄ Fettalkoholsulfate (k = 11-13 in Formel A-2a).

Weiter bevorzugte Zusammensetzungen enthalten bezogen auf die Gesamtmenge der Zusammensetzungen 1,0 bis 25,0 Gew.-%, vorzugsweise 2,5 bis 20,5 Gew.-%, weiter bevorzugt 5,0 bis 20,0 Gew.-% Tensid aus der Gruppe umfassend C₉₋₁₃-Alkylbenzolsulfonate, Olefinsulfonate, C₁₂₋₁₈-Alkansulfonate Estersulfonate, Alk(en)ylsulfate, und Mischungen daraus (insbesondere der Gruppe der C₉₋₁₃-Alkylbenzolsulfonate, bevorzugt der Gruppe gemäß Formel (A2)).

Bei den Sulfonattensiden (A = chemische Bindung), welche gegenüber den Sulfattensiden obiger Formel bevorzugt sind, steht R³ vorzugsweise für einen linearen oder verzweigten unsubstituierten Alkylarylrest. Auch hier steht X für ein einwertiges Kation oder den n-ten Teil eines n-wertigen Kations, bevorzugt sind dabei die Alkalimetallionen und darunter Na⁺ oder K⁺, wobei Na⁺ äußerst bevorzugt ist. Weitere Kationen X+ können ausgewählt sein aus NH₄⁺, ½ Zn²⁺,½ Mg²⁺,½ Ca²⁺,½ Mn²⁺, und deren Mischungen.

Solche äußerst bevorzugten Tenside sind ausgewählt aus linearen oder verzweigten Alkylbenzolsulfonaten der Formel A-3 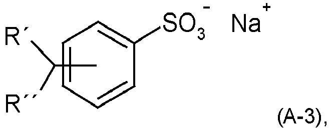 in der R' und R" zusammen 9 bis 19, vorzugsweise 11 bis 15 und insbesondere 11 bis 13 C-Atome enthalten. Ein ganz besonders bevorzugter Vertreter lässt sich durch die Formel A-3a beschreiben:

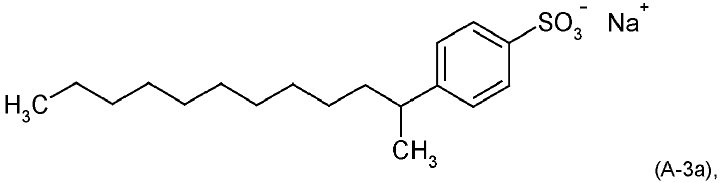

Es hat sich für die Kaltwaschleistung als vorteilhaft erwiesen, wenn die Zusammensetzungen als anionisches Tensid zusätzlich Seife(n) enthalten. Seifen sind die wasserlöslichen Natrium- oder Kaliumsalze der gesättigten und ungesättigten Fettsäuren mit 10 bis 20 Kohlenstoffatomen, der Harzsäuren des Kolophoniums (gelbe Harzseifen) und der Naphthensäuren, die als feste oder halbfeste Gemische in der Hauptsache für Wasch- und Reinigungszwecke verwendet werden. Natrium- oder Kaliumsalze der gesättigten und ungesättigten Fettsäuren mit 10 bis 20 Kohlenstoffatomen, insbesondere mit 12 bis 18 Kohlenstoffatomen, sind gemäß Erfindung bevorzugte Seifen. Besonders bevorzugte Zusammensetzungen sind dabei dadurch gekennzeichnet, dass sie - bezogen auf ihr Gewicht - 0,1 bis 6 Gew.-%, besonders bevorzugt 0,2 bis 4,5 Gew.-%, ganz besonders bevorzugt 0,3 bis 4,1 Gew.-% Seife(n) enthalten.

Zur verbesserten Lösung der technischen Aufgabe ist es erfindungsgemäß ganz besonders bevorzugt, eine Kombination aus
- mindestens einem Fettalkoholethersulfat der Formel A-1 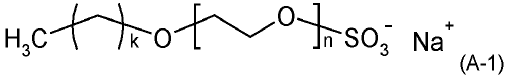 mit k = 11 bis 19, n = 2, 3, 4, 5, 6, 7 oder 8 (besonders bevorzugte Vertreter sind Na-C₁₂₋₁₄ Fettalkoholethersulfate mit 2 EO (k = 11-13, n = 2 in Formel A-1), und
- mindestens einem linearen oder verzweigten Alkylbenzolsulfonaten der Formel A-3 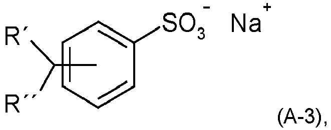 in der R' und R" zusammen 9 bis 19, vorzugsweise 11 bis 15 und insbesondere 11 bis 13 C-Atome enthalten (insbesondere der obigen Formel (A-3a)),
in den erfindungsgemäßen Zusammensetzungen zu verwenden. Bevorzugte Zusammensetzungen dieser Ausführungsform sind dabei optional bevorzugt dadurch gekennzeichnet, dass sie eine oder mehrere Seifen enthält (bevorzugt - bezogen auf ihr Gewicht - 0,1 bis 6 Gew.-%, besonders bevorzugt 0,2 bis 4,5 Gew.-%, ganz besonders bevorzugt 0,3 bis 4,1 Gew.-% Seife(n)).

Zusätzlich zu dem oder den anionischen Tensid(en) oder an deren Stelle können die erfindungsgemäß eingesetzten Zusammensetzungen nichtionische(s) Tensid(e) enthalten.

Mit besonderem Vorzug enthalten die Zusammensetzungen mindestens ein nichtionisches Tensid aus der Gruppe der Fettalkoholethoxylate, da diese Tenside auch bei niedrigen Waschtemperaturen leistungsstarke Zusammensetzungen ergeben und im Falle flüssiger Zubereitungen exzellente Kältestabilität aufweisen.

Demnach enthalten bevorzugte Zusammensetzungen zusätzlich mindestens ein nichtionisches Tensid der Formel

R²-O-(AO)ₘ-H,

in der
- R²: für einen linearen oder verzweigten, substituierten oder unsubstituierten Alkyl-, Aryl- oder Alkylarylrest,
- AO: für eine Ethylenoxid- (EO) oder Propylenoxid- (PO) Gruppierung,
- m: für ganze Zahlen von 1 bis 50 stehen.

In der vorstehend genannten Formel steht R² für einen linearen oder verzweigten, substituierten oder unsubstituierten Alkyl-, Aryl- oder Alkylarylrest, vorzugsweise für einen linearen, unsubstituierten Alkylrest, besonders bevorzugt für einen Fettalkoholrest. Bevorzugte Reste R² sind ausgewählt aus Decyl-, Undecyl-, Dodecyl-, Tridecyl-, Tetradecyl, Pentadecyl-, Hexadecyl-, Heptadecyl-, Octadecyl-, Nonadecyl-, Eicosylresten und deren Mischungen, wobei die Vertreter mit gerader Anzahl an C-Atomen bevorzugt sind. Besonders bevorzugte Reste R² sind abgeleitet von C₁₂-C₁₈-Fettalkoholen, beispielsweise von Kokosfettalkohol, Talgfettalkohol, Lauryl-, Myristyl-, Cetyl- oder Stearylalkohol oder von C₁₀-C₂₀-Oxoalkoholen.

AO steht für eine Ethylenoxid- (EO) oder Propylenoxid- (PO) Gruppierung, vorzugsweise für eine Ethylenoxidgruppierung. Der Index m steht für eine ganze Zahl von 1 bis 50, vorzugsweise von 1 bis 20 und insbesondere von 2 bis 10. Ganz besonders bevorzugt steht m für die Zahlen 2, 3, 4, 5, 6, 7 oder 8.

Zusammenfassend sind besonders bevorzugte Tenside ausgewählt aus Fettalkoholethoxylaten der Formel C-1 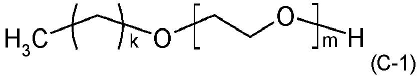 mit k = 11 bis 19, m = 2, 3, 4, 5, 6, 7 oder 8. Ganz besonders bevorzugte Vertreter sind C₁₂₋₁₈ Fettalkohole mit 7 EO (k = 11-17, m = 7 in Formel C-1).

Insbesondere bevorzugte Zusammensetzungen enthalten nichtionische Tenside in bestimmten Mengen. Äußerst bevorzugte erfindungsgemäße Zusammensetzungen sind dadurch gekennzeichnet, dass die Gesamtmenge an nichtionischen Tensiden bezogen auf das Gewicht der Zusammensetzungen 1,0 bis 25 Gew.-%, vorzugsweise 2,5 bis 20,0 Gew.-%, weiter bevorzugt 5,0 bis 18,0 Gew.-% beträgt.

Weiter bevorzugte Zusammensetzungen enthalten bezogen auf die Gesamtmenge der Zusammensetzungen 1,0 bis 25 Gew.-%, vorzugsweise 2,5 bis 20,0 Gew.-%, weiter bevorzugt 5,0 bis 18,0 Gew.-% Fettalkoholethoxylat(e) (insbesondere der Formel (C-1)).

Bevorzugte Zusammensetzungen enthalten auf das Gesamtgewicht der Zusammensetzung bezogen
- anionisches Tensid in einer Gesamtmenge von 1,0 bis 35,0 Gew.-%, bevorzugt von 5,0 bis 30,0 Gew.-%, besonders bevorzugt von 10,0 bis 25,0 Gew.-%, und
- nichtionisches Tensid in einer Gesamtmenge von 1,0 bis 25 Gew.-%, vorzugsweise 2,5 bis 20,0 Gew.-%, weiter bevorzugt 5,0 bis 18,0 Gew.-%.

Hierbei ist es wiederum bevorzugt, die bevorzugten Tenside (am bevorzugtesten in den als bevorzugt gekennzeichneten Mengen) einzusetzen. Die Menge der speziellen anionischen und nichtionischen Tenside ist in diesem Falle so zu wählen, dass die zuvor definierte Gesamtmenge an anionischem Tensid und die zuvor definierte Gesamtmenge an nichtionischem Tensid eingehalten wird.

Gemäß einer besonders bevorzugten Ausführungsform sind solche Zusammensetzungen bevorzugt, die
i) mindestens ein anionisches Tensid der Formel R¹-O-(AO)ₙ-SO₃⁻ X⁺, und
ii) mindestens ein anionisches Tensid der Formel A-3 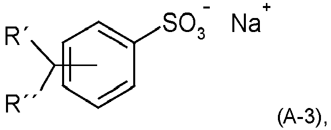 und
iii) mindestens ein nichtionisches Tensid der Formel R²-O-(AO)ₘ-H, enthalten, in denen
   - R¹: für einen linearen oder verzweigten, substituierten oder unsubstituierten Alkyl-, Aryl- oder Alkylarylrest,
   - R' und R": zusammen 9 bis 19, vorzugsweise 11 bis 15 und insbesondere 11 bis 13 C-Atome enthalten,
   - AO: unabhängig voneinander für eine Ethylenoxid- (EO) oder Propylenoxid- (PO) Gruppierung,
   - n, m: unabhängig voneinander für ganze Zahlen von 1 bis 50,
   - X: für ein einwertiges Kation oder den n-ten Teil eines n-wertigen Kations stehen.

Die Tenside i) und ii) wurden weiter oben als bevorzugte Tenside a) mit den Formeln (A-1) und (A-3a) beschrieben, das Tensid iii) als bevorzugtes Tensid mit der Formel (C-1). Bevorzugte Zusammensetzungen dieser Ausführungsform sind dabei wiederum dadurch gekennzeichnet, dass sie zusätzlich mindesten eine Seife enthalten.

Gemäß einer ganz besonders bevorzugten Ausführungsform sind solche Zusammensetzungen bevorzugt, die bezogen auf das Gewicht der Zusammensetzung
i) in einer Gesamtmenge von 1,0 bis 15 Gew.-%, vorzugsweise 2,5 bis 12,5 Gew.-%, weiter bevorzugt 5,0 bis 10,0 Gew.-% mindestens ein anionisches Tensid der Formel R¹-O-(AO)ₙ-SO₃⁻ X⁺, und
ii) in einer Gesamtmenge von 1,0 bis 25 Gew.-%, vorzugsweise 2,5 bis 20,5 Gew.-%, weiter bevorzugt 5,0 bis 20,0 Gew.-% mindestens ein anionisches Tensid der Formel A-3 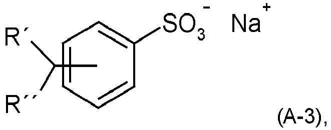 und
iii) in einer Gesamtmenge von 1,0 bis 25 Gew.-%, vorzugsweise 2,5 bis 20,0 Gew.-%, weiter bevorzugt 5,0 bis 18,0 Gew.-% mindestens ein nichtionisches Tensid der Formel

   R²-O-(AO)ₘ-H,
enthalten, in denen
- R¹: für einen linearen oder verzweigten, substituierten oder unsubstituierten Alkyl-, Aryl- oder Alkylarylrest,
- R' und R": zusammen 9 bis 19, vorzugsweise 11 bis 15 und insbesondere 11 bis 13 C-Atome enthalten,
- AO: unabhängig voneinander für eine Ethylenoxid- (EO) oder Propylenoxid- (PO) Gruppierung,
- n, m: unabhängig voneinander für ganze Zahlen von 1 bis 50,
- X: für ein einwertiges Kation oder den n-ten Teil eines n-wertigen Kations stehen,

Bevorzugte Zusammensetzungen dieser Ausführungsform sind dabei wiederum dadurch gekennzeichnet, dass sie - bezogen auf ihr Gewicht - 0,1 bis 6 Gew.-%, besonders bevorzugt 0,2 bis 4,5 Gew.-%, ganz besonders bevorzugt 0,3 bis 4,1 Gew.-% Seife(n) enthalten.

Die erfindungsgemäße Zusammensetzung kann als Lösemittel Wasser enthalten.

Bei den Waschmitteln handelt es sich um wasserarme Waschmittel, wobei der Gehalt an Wasser weniger als 30 Gew.-%, besonders bevorzugt weniger als 10 Gew.-%, ganz besonders bevorzugt weniger als 8 Gew.-%, jeweils bezogen auf das gesamte flüssige Waschmittel, beträgt.

Das Warmformverfahren schließt im Allgemeinen das Formen einer ersten Lage aus einem wasserlöslichen Folienmaterial zum Bilden von mindestens einer Ausbuchtung zum Aufnehmen jeweils mindestens einer Zusammensetzung darin, Einfüllen der Zusammensetzung in die jeweilige Ausbuchtung, Bedecken der mit der Zusammensetzung gefüllten Ausbuchtungen mit einer zweiten Lage aus einem wasserlöslichen Folienmaterial und Versiegeln der ersten und zweiten Lagen miteinander zumindest um die Ausbuchtungen herum, ein.

Die wasserlösliche Folie enthält bevorzugt mindestens ein wasserlösliches Polymer als Folienmaterial. Die Umhüllung der erfindungsgemäßen flüssigen Zusammensetzung kann aus einer oder aus zwei oder mehr Lagen aus dem wasserlöslichen Folienmaterial gebildet werden. Das wasserlösliche Folienmaterial der ersten Lage und der weiteren Lagen, falls vorhanden, kann gleich oder unterschiedlich sein.

Es ist bevorzugt, dass das wasserlösliche Folienmaterial Polyvinylalkohol oder ein Polyvinylalkoholcopolymer enthält.

Geeignete wasserlösliche Folien basieren bevorzugt auf einem Polyvinylalkohol oder einem Polyvinylalkoholcopolymer, dessen Molekulargewicht jeweils im Bereich von 10.000 bis 1.000.000 gmol⁻¹, vorzugsweise von 20.000 bis 500.000 gmol⁻¹, besonders bevorzugt von 30.000 bis 100.000 gmol-' und insbesondere von 40.000 bis 80.000 gmol-' liegt.

Die Herstellung von Polyvinylalkohol geschieht üblicherweise durch Hydrolyse von Polyvinylacetat, da der direkte Syntheseweg nicht möglich ist. Ähnliches gilt für Polyvinylalkoholcopolymere, die aus entsprechend aus Polyvinylacetatcopolymeren hergestellt werden. Bevorzugt ist, wenn wenigstens eine Lage des wasserlöslichen Materials einen Polyvinylalkohol umfasst, dessen Hydrolysegrad 70 bis 100 Mol-%, vorzugsweise 80 bis 90 Mol-%, besonders bevorzugt 81 bis 89 Mol-% und insbesondere 82 bis 88 Mol-% ausmacht.

Dem wasserlöslichen Folienmaterial können zusätzlich Polymere, ausgewählt aus der Gruppe umfassend Acrylsäure-haltige Polymere, Polyacrylamide, Oxazolin-Polymere, Polystyrolsulfonate, Polyurethane, Polyester, Polyether Polymilchsäure, und/oder Mischungen der vorstehenden Polymere, zugesetzt sein.

Bevorzugte Polyvinylalkoholcopolymere umfassen neben Vinylalkohol Dicarbonsäuren als weitere Monomere. Geeignete Dicarbonsäure sind Itaconsäure, Malonsäure, Bernsteinsäure und Mischungen daraus, wobei Itaconsäure bevorzugt ist.

Ebenso bevorzugte Polyvinylalkoholcopolymere umfassen neben Vinylalkohol eine ethylenisch ungesättige Carbonsäure, deren Salz oder deren Ester. Besonders bevorzugt enthalten solche Polyvinylalkoholcopolymere neben Vinylalkohol Acrylsäure, Methacrylsäure, Acrylsäureester, Methacrylsäureester oder Mischungen daraus.

Geeignete wasserlösliche Folien zur Herstellung der wasserlöslichen Portion sind Folien, die unter der Bezeichnung Monosol M8630 von MonoSol LLC vertrieben werden. Andere geeignete Folien umfassen Folien mit der Bezeichnung Solublon^{®} PT, Solublon^{®} KA, Solublon^{®} KC oder Solublon^{®} KL von der Aicello Chemical Europe GmbH oder die Folien VF-HP von Kuraray, oder HiSelon SH2312 der Firma Nippon Gohsei.

Erfindungsgemäß besonders bevorzugte Portionen enthalten in der erfindungsgemäß darin konfektionierten flüssigen Zusammensetzung jeweils bezogen auf das Gesamtgewicht der flüssigen Zusammensetzung jeweils eine Gesamtmenge von
- 30 bis 40 Gew.-% mindestens eines anionischen Tensids und
- 18 bis 28 Gew.-% mindestens eines nichtionischen Tensids.

Erfindungsgemäß ganz besonders bevorzugte erfindungsgemäße flüssige Zusammensetzungen zur Konfektionierung in einer besagten Portion enthalten als Tensid mindestens eine der bevorzugten Kombinationen (A) bis (D):
(A) Jeweils bezogen auf das Gesamtgewicht der flüssigen Zusammensetzung jeweils eine Gesamtmenge von
   - 25 bis 60 Gew.-% mindestens eines anionischen Tensids, wobei als anionisches Tensid mindestens ein C₉₋₁₃-Alkylbenzolsulfonat enthalten ist, und
   - 2 bis 35 Gew.-% mindestens eines nichtionischen Tensids, wobei als nichtionisches Tensid mindestens ein alkoxylierter Alkohol mit 8 bis 18 Kohlenstoffatomen und durchschnittlich 4 bis 12 Mol Ethylenoxid (EO) pro Mol Alkohol enthalten ist.
(B) Jeweils bezogen auf das Gesamtgewicht der flüssigen Zusammensetzung jeweils eine Gesamtmenge von
   - 25 bis 60 Gew.-% mindestens eines anionischen Tensids, wobei als anionisches Tensid mindestens 25 bis 60 Gew.-% mindestens eines C₉₋₁₃-Alkylbenzolsulfonats enthalten ist, und
   - 2 bis 35 Gew.-% mindestens eines nichtionischen Tensids, wobei als nichtionisches Tensid mindestens 2 bis 35 Gew.-% mindestens eines alkoxylierten Alkohols mit 8 bis 18 Kohlenstoffatomen und durchschnittlich 4 bis 12 Mol Ethylenoxid (EO) pro Mol Alkohol enthalten ist.
(C) Jeweils bezogen auf das Gesamtgewicht der flüssigen Zusammensetzung jeweils eine Gesamtmenge von
   - 30 bis 40 Gew.-% mindestens eines anionischen Tensids, wobei als anionisches Tensid mindestens ein C₉₋₁₃-Alkylbenzolsulfonat enthalten ist, und
   - 18 bis 28 Gew.-% mindestens eines nichtionischen Tensids, wobei als nichtionisches Tensid mindestens ein alkoxylierter Alkohol mit 8 bis 18 Kohlenstoffatomen und durchschnittlich 4 bis 12 Mol Ethylenoxid (EO) pro Mol Alkohol enthalten ist.
(D) Jeweils bezogen auf das Gesamtgewicht der flüssigen Zusammensetzung jeweils eine Gesamtmenge von
   - 30 bis 40 Gew.-% mindestens eines anionischen Tensids, wobei als anionisches Tensid mindestens 15 bis 30 Gew.-% mindestens eines C₉₋₁₃-Alkylbenzolsulfonats enthalten ist, und
   - 18 bis 28 Gew.-% mindestens eines nichtionischen Tensids, wobei als nichtionisches Tensid mindestens 15 bis 28 Gew.-% mindestens eines alkoxylierten Alkohols mit 8 bis 18 Kohlenstoffatomen und durchschnittlich 4 bis 12 Mol Ethylenoxid (EO) pro Mol Alkohol enthalten ist.

Neben den erfindungsgemäß zwingend vorhandenen Komponenten können der erfindungsgemäßen flüssigen Zusammensetzung nichtwässrige Lösungsmittel zugesetzt werden. Geeignete nichtwässrige Lösungsmittel umfassen ein- oder mehrwertige Alkohole, Alkanolamine oder Glykolether, sofern sie im angegebenen Konzentrationsbereich mit Wasser mischbar sind. Vorzugsweise werden die Lösungsmittel ausgewählt aus Ethanol, n-Propanol, i-Propanol, Butanolen, Glykol, Propandiol, Butandiol, Methylpropandiol, Glycerin, Diglykol, Propyldiglycol, Butyldiglykol, Hexylenglycol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethylether, Propylenglykolethylether, Propylenglykolpropylether, Dipropylenglykolmonomethylether, Dipropylenglykolmonoethylether, Methoxytriglykol, Ethoxytriglykol, Butoxytriglykol, 1-Butoxyethoxy-2-propanol, 3-Methyl-3-methoxybutanol, Propylen-glykol-t-butylether, Di-n-octylether sowie Mischungen dieser Lösungsmittel. Es ist allerdings bevorzugt, dass die erfindungsgemäße Zusammensetzung einen Alkohol, insbesondere Ethanol und/oder Glycerin und/oder 1,2-Propandiol, in Mengen von 0,5 und 5 Gew.-%, bezogen auf die gesamte Zusammensetzung enthält.

Die erfindungsgemäße flüssige Zusammensetzung enthält zwingend mindestens eine spezielle α-Amylase als Enzym.

Folgende Begriffe und Definitionen gelten zusätzlich im Rahmen der vorliegenden Anmeldung mit Blick auf die Definition von Enzymen.

"Variante" ist auf der Ebene der Proteine der zu "Mutante" entsprechende Begriff auf der Ebene der Nukleinsäuren. Bei den Vorgänger- oder Ausgangsmolekülen kann es sich um Wildtypenzyme handeln, das heißt solche, die aus natürlichen Quellen erhältlich sind. Es kann sich auch um Enzyme handeln, die an sich bereits Varianten darstellen, das heißt gegenüber den Wildtypmolekülen bereits verändert worden sind. Darunter sind beispielsweise Punktmutanten, solche mit Änderungen der Aminosäuresequenz, über mehrere Positionen oder längere zusammenhängende Bereiche, oder auch Hybridmoleküle zu verstehen, die aus einander ergänzenden Abschnitten verschiedener Wildtyp Enzyme zusammengesetzt sind.

Grundsätzlich sind die mit der vorliegenden Anmeldung bezeichneten erfindungsgemäßen Aminosäureaustausche nicht darauf beschränkt, daß sie die einzigen Austausche sind, in denen sich die betreffende Variante von dem Ausgangsmolekül unterscheidet. Es ist im Stand der Technik bekannt, daß sich die vorteilhaften Eigenschaften einzelner Punktmutationen einander ergänzen können. Somit umfassen Ausführungsformen der vorliegenden Erfindung alle Varianten, die neben anderen Austauschen gegenüber dem Ausgangsmolekül auch die erfindungsgemäßen Austausche aufweisen.

Ferner spielt es prinzipiell keine Rolle, in welcher Reihenfolge die betreffenden Aminosäureaustausche vorgenommen worden sind, das heißt ob eine entsprechende Punktmutante erfindungsgemäß weiterentwickelt wird oder zunächst beispielsweise aus einem Ausgangsmolekül eine erfindungsgemäße Variante erzeugt wird, die entsprechend anderer im Stand der Technik zu findender Lehren weiterentwickelt wird. Es können auch gleichzeitig in einem Mutageneseansatz mehrere Austausche vorgenommen werden, etwa erfindungsgemäße und andere zusammen.

Die Bestimmung der Identität von Nukleinsäure- oder Aminosäuresequenzen erfolgt erfindungsgemäß durch einen Sequenzvergleich. Solch ein Vergleich erfolgt dadurch, dass ähnliche Abfolgen in den Nukleotidsequenzen oder Aminosäuresequenzen einander zugeordnet werden. Dieser Sequenzvergleich erfolgt vorzugsweise basierend auf dem im Stand der Technik etablierten und üblicherweise genutzten BLAST-Algorithmus (vgl. beispielsweise Altschul, S. F., Gish, W., Miller, W., Myers, E. W. & Lipman, D. J. (1990) "Basic local alignment search tool." J. Mol. Biol. 215:403-410 , und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of Protein database search programs"; Nucleic Acids Res., 25, S. 3389-3402 ) und geschieht prinzipiell dadurch, dass ähnliche Abfolgen von Nukleotiden oder Aminosäuren in den Nukleinsäure- bzw. Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden üblicherweise mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen bzw. Algorithmen basieren. Häufig genutzt werden beispielsweise Clustal (vgl. beispielsweise Chenna et al. (2003): Multiple sequence alignment with the Clustal series of programs. Nucleic Acid Research 31, 3497-3500) oder T-Coffee (vgl. beispielsweise Notredame et al. (2000): T-Coffee: A novel method for multiple sequence alignments. J. Mol. Biol. 302, 205-217) sowie BLAST oder FASTA für die Datenbanksuche, beziehungsweise Programme, die auf diesen Programmen bzw. Algorithmen basieren. Im Rahmen der vorliegenden Erfindung werden Sequenzvergleiche und Alignments bevorzugt mit dem Computer-Programm Vector NTI^{®} Suite 10.3 (Invitrogen Corporation, 1600 Faraday Avenue, Carlsbad, Kalifornien, USA) mit den vorgegebenen Default-Parametern erstellt.

Solch ein Vergleich erlaubt eine Aussage über die Ähnlichkeit der verglichenen Sequenzen zueinander. Sie wird üblicherweise in Prozent Identität, das heißt dem Anteil der identischen Nukleotide oder Aminosäurereste an denselben bzw. in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch in Prozent Homologie oder Prozent Ähnlichkeit angegeben sein. Identitäts- und/oder Homologieangaben können über ganze Polypeptide oder Gene oder nur über einzelne Bereiche getroffen werden. Homologe bzw. identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide bzw. Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- bzw. Homologieangaben in der vorliegenden Anmeldung aber auf die Gesamtlänge der jeweils angegebenen Nukleinsäure- oder Aminosäuresäuresequenz.

Unter Fragmenten werden alle Polypeptide, Proteine oder Peptide verstanden, die kleiner sind als entsprechende Vergleichsproteine oder solche, die vollständig translatierten Genen entsprechen, und beispielsweise auch synthetisch erhalten werden können. Aufgrund ihrer Aminosäuresequenzen können sie den betreffenden vollständigen Vergleichsproteinen zugeordnet werden. Sie können beispielsweise gleiche räumliche Strukturen annehmen oder proteolytische Aktivitäten oder Teilaktivitäten ausüben, wie beispielsweise die Komplexierung eines Substrats. Fragmente und Deletionsvarianten von Ausgangsproteinen sind prinzipiell gleichartig; während Fragmente eher kleinere Bruchstücke darstellen, fehlen den Deletionsmutanten eher nur kurze Bereiche (unter Umständen nur ein oder mehrere Aminosäuren). So ist es beispielsweise möglich, an den Termini oder in den Loops des Enzyms weitere einzelne Aminosäuren zu deletieren, ohne dass dadurch die enzymatische Aktivität verloren oder vermindert wird.

Alle Mengenangaben von Enzymen beziehen sich auf aktives Protein. Die Proteinkonzentration kann mit Hilfe bekannter Methoden, zum Beispiel dem BCA-Verfahren oder dem Biuret-Verfahren bestimmt werden. Die Bestimmung der Aktivproteinkonzentration kann diesbezüglich über eine Titration der aktiven Zentren unter Verwendung eines geeigneten irreversiblen Inhibitors (für Proteasen beispielsweise Phenylmethylsulfonylfluorid (PMSF)) und Bestimmung der Restaktivität (vgl. M. Bender et al., J. Am. Chem. Soc. 88, 24 (1966), S. 5890-5913) erfolgen.

Zusätzlich zu den vorstehend erläuterten Aminosäureveränderungen können erfindungs¬gemäße Enzyme weitere Aminosäureveränderungen, insbesondere Aminosäure-Substitutionen, Insertionen oder Deletionen, aufweisen. Solche Enzyme sind beispielsweise durch gezielte genetische Veränderung, d.h. durch Mutageneseverfahren, weiterentwickelt und für bestimmte Einsatzzwecke oder hinsichtlich spezieller Eigenschaften (beispielsweise hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Enzyme oder anderer Polypeptide genutzt werden.

Das Ziel ist es, in die bekannten Moleküle gezielte Mutationen wie Substitutionen, Insertionen oder Deletionen einzuführen, um beispielsweise die Reinigungsleistung von erfindungsgemäßen Enzymen zu verbessern. Hierzu können insbesondere die Oberflächenladungen und/oder der isoelektrische Punkt der Moleküle und dadurch ihre Wechselwirkungen mit dem Substrat verändert werden. So kann beispielsweise die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität der Amylase erhöht und dadurch ihre Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierte Amylase, zum Beispiel hinsichtlich ihrer Stabilität gegenüber Tensiden und/oder Bleichmitteln und/oder anderen Komponenten, kann daher im Rahmen der Erfindung zusätzlich weiterentwickelt sein.

Für die Beschreibung von Substitutionen, die genau eine Aminosäureposition betreffen (Aminosäureaustausche), wird folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere Austausche innerhalb derselben Polypeptidkette werden durch Schrägstriche voneinander getrennt. Bei Insertionen sind nach der Sequenzposition zusätzliche Aminosäuren benannt. Bei Deletionen ist die fehlende Aminosäure durch ein Symbol, beispielsweise einen Stern oder einen Strich, ersetzt. Beispielsweise beschreibt A95G die Substitution von Alanin an Position 95 durch Glycin, A95AG die Insertion von Glycin nach der Aminosäure Alanin an Position 95 und A95* die Deletion von Alanin an Position 95. Diese Nomenklatur ist dem Fachmann auf dem Gebiet der Enzymtechnologie bekannt.

Die erfindungsgemäße flüssige Zusammensetzung enthält zwingend mindestens eine α-Amylase, die zu der in SEQ ID NO.1 angegeben Sequenz über deren Gesamtlänge zu mindestens 89% und zunehmend bevorzugt zu mindestens 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99%, 99,5% und bis zu 100% identisch ist und in der Zählung gemäß SEQ ID NO. 1 an einer oder mehreren der Positionen 180, 181, 182, 183 und 184 Deletionen aufweist.

Aminosäurepositionen, die im Rahmen der vorliegenden Erfindung mit der Formulierung "Zählung gemäß SEQ ID NO. 1" angegeben werden, verstehen sich folgendermaßen: Die weiteren Aminosäurepositionen werden durch ein Alignment der Aminosäuresequenz einer erfindungsgemäßen Amylase mit der Aminosäuresequenz, wie sie in SEQ ID NO. 1 angegeben ist, definiert. Weiterhin richtet sich die Zuordnung der Positionen nach dem reifen (maturen) Protein. Diese Zuordnung ist insbesondere auch anzuwenden, wenn die Aminosäuresequenz eines erfindungsgemäßen Proteins eine höhere Zahl von Aminosäureresten umfasst als die Amylase in SEQ ID NO. 1. Ausgehend von den genannten Positionen in der Aminosäuresequenz sind die Veränderungspositionen in einer erfindungsgemäßen Amylase diejenigen, die eben diesen Positionen in einem Alignment zugeordnet sind.

Besonders bevorzugt ist eine Deletion von zwei Positionen ausgewählt aus 180+181, 181+182, 182+183 und 183+184, ganz besonders bevorzugt sind Deletionen an den Positionen 183+184 in der Zählung gemäß SEQ ID NO. 1, insbesondere bevorzugt die Deletionen H183* + G184*.

Vorzugsweise weist die α-Amylase der erfindungsgemäßen flüssigen Zusammensetzung in der Zählung gemäß SEQ ID NO. 1 weiterhin eine Aminosäuresubstitution an einer oder mehreren der Positionen 405, 421, 422 und 428 auf. Besonders bevorzugt sind die Substitutionen I405L; A421H, A422P und A428T.

In einer besonders bevorzugten Ausführungsform weist die α-Amylase der erfindungsgemäßen flüssigen Zusammensetzung in der Zählung gemäß SEQ ID NO. 1 die Deletionen H183* + G184* und zusätzlich die Substitutionen I405L, A421H, A422P und A428T auf.

Ein weiterer Gegenstand der vorliegenden Erfindung ist daher ein Wasch und-Reinigungsmittel enthaltend eine Kombination aus einer α-Amylase und einer Protease, wobei die α-Amylase dadurch gekennzeichnet ist, dass sie aus einer erfindungsgemäßen Amylase als Ausgangsmolekül durch ein- oder mehrfache konservative Aminosäuresubstitution erhältlich ist. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z. B. der Austausch eines unpolaren Aminosäurerestes gegen einen anderen unpolaren Aminosäurerest. Konservative Aminosäuresubstitutionen im Rahmen der Erfindung umfassen beispielsweise: G=A=S, I=V=L=M, D=E, N=Q, K=R, Y=F, S=T, G=A=I=V=L=M=Y=F=W=P=S=T.

Eine weitere Ausführungsform der vorliegenden Erfindung ist daher eine besagte flüssige Zusammensetzung, insbesondere zur Wäsche oder Reinigung von Textilien, enthaltend mindestens eine α-Amylase, wobei die α-Amylase dadurch gekennzeichnet ist, dass sie
(a)
   (i) aus einer α-Amylase als Ausgangsmolekül durch Fragmentierung, Deletions-, Insertions- oder Substitutionsmutagenese erhältlich ist, und
   (ii) besagtes Ausgangsmolekül zu der in SEQ ID NO.1 angegebenen Sequenz über deren Gesamtlänge zu mindestens 89% und zunehmend bevorzugt zu mindestens 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99%, 99,5% und bis zu 100% identisch ist und in der Zählung gemäß SEQ ID NO. 1 an einer oder mehreren der Positionen 180, 181, 182, 183 und 184 Deletionen aufweist, und
(b) eine Aminosäuresequenz umfasst, die über eine Länge von mindestens 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 475, 480, 482, 484 oder 485 zusammenhängenden Aminosäuren mit dem Ausgangsmolekül übereinstimmt.

So ist es beispielsweise möglich, an den Termini oder in den Loops des Enzyms (Ausgangsmolekül) einzelne Aminosäuren zu deletieren, ohne daß dadurch die proteolytische Aktivität verloren oder vermindert wird. Ferner kann durch derartige Fragmentierung, Deletions-, Insertions- oder Substitutionsmutagenese beispielsweise auch die Allergenizität betreffender Enzyme gesenkt und somit insgesamt ihre Einsetzbarkeit verbessert werden. Vorteilhafterweise behalten die Enzyme auch nach der Mutagenese ihre proteolytische Aktivität, d.h. ihre proteolytische Aktivität entspricht mindestens derjenigen des Ausgangsenzyms. Auch Substitutionen können vorteilhafte Wirkungen zeigen. Sowohl einzelne wie auch mehrere zusammenhängende Aminosäuren können gegen andere Aminosäuren ausgetauscht werden.

Eine erfindungsgemäße Amylase kann zusätzlich stabilisiert sein, insbesondere durch eine oder mehrere Mutationen, beispielsweise Substitutionen, oder durch Kopplung an ein Polymer. Denn eine Erhöhung der Stabilität bei der Lagerung und/oder während des Einsatzes, beispielsweise beim Waschprozess, führt dazu, daß die enzymatische Aktivität länger anhält und damit die Reinigungsleistung verbessert wird. Grundsätzlich kommen alle im Stand der Technik beschriebenen und/oder zweckmäßigen Stabilisierungsmöglichkeiten in Betracht. Bevorzugt sind solche Stabilisierungen, die über Mutationen des Enzyms selbst erreicht werden, da solche Stabilisierungen im Anschluss an die Gewinnung des Enzyms keine weiteren Arbeitsschritte erfordern. Hierfür geeignete Sequenzveränderungen sind aus dem Stand der Technik bekannt.

Weitere Möglichkeiten der Stabilisierung sind z. B.:
- Veränderung der Bindung von Metallionen, insbesondere der Calcium-Bindungsstellen, beispielsweise durch Austauschen von einer oder mehreren der an der Calcium-Bindung beteiligten Aminosäure(n) gegen eine oder mehrere negativ geladene Aminosäuren und/oder durch Einführen von Sequenzveränderungen in mindestens einer der Folgen der beiden Aminosäuren Arginin/Glycin;
- Schutz gegen den Einfluss von denaturierenden Agentien wie Tensiden durch Mutationen, die eine Veränderung der Aminosäuresequenz auf oder an der Oberfläche des Proteins bewirken;
- Austausch von Aminosäuren, die nahe dem N-Terminus liegen, gegen solche, die vermutlich über nicht-kovalente Wechselwirkungen mit dem Rest des Moleküls in Kontakt treten und somit einen Beitrag zur Aufrechterhaltung der globulären Struktur leisten.

Bevorzugte Ausführungsformen sind solche, bei denen das Enzym auf mehrere Arten stabilisiert wird, da mehrere stabilisierende Mutationen additiv oder synergistisch wirken.

Eine weiterere Ausführungsform der Erfindung ist eine besagte flüssige Zusammensetzung, insbesondere zur Wäsche oder Reinigung von Textilien, enthaltend mindestens eine α-Amylase wie vorstehend beschrieben, die dadurch gekennzeichnet ist, daß sie mindestens eine chemische Modifikation aufweist. Eine Amylase mit einer solchen Veränderung wird als Derivat bezeichnet, d.h. die Amylase ist derivatisiert.

Unter Derivaten werden im Sinne der vorliegenden Anmeldung demnach solche Proteine verstanden, deren reine Aminosäurekette chemisch modifiziert worden ist. Solche Derivatisierungen können beispielsweise in vivo durch die Wirtszelle erfolgen, die das Protein exprimiert. Diesbezüglich sind Kopplungen niedrigmolekularer Verbindungen wie von Lipiden oder Oligosacchariden besonders hervorzuheben. Derivatisierungen können aber auch in vitro durchgeführt werden, etwa durch die chemische Umwandlung einer Seitenkette einer Aminosäure oder durch kovalente Bindung einer anderen Verbindung an das Protein. Beispielsweise ist die Kopplung von Aminen an Carboxylgruppen eines Enzyms zur Veränderung des isoelektrischen Punkts möglich. Eine solche andere Verbindung kann auch ein weiteres Protein sein, das beispielsweise über bifunktionelle chemische Verbindungen an ein erfindungsgemäßes Protein gebunden wird. Ebenso ist unter Derivatisierung die kovalente Bindung an einen makromolekularen Träger zu verstehen, oder auch ein nichtkovalenter Einschluss in geeignete makromolekulare Käfigstrukturen. Derivatisierungen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Derartige Modifikationen können ferner die Stabilität oder die enzymatische Aktivität beeinflussen. Sie können ferner auch dazu dienen, die Allergenizität und/oder Immunogenizität des Proteins herabzusetzen und damit beispielsweise dessen Hautverträglichkeit zu erhöhen. Beispielsweise können Kopplungen mit makromolekularen Verbindungen, beispielsweise Polyethylenglykol, das Protein hinsichtlich der Stabilität und/oder Hautverträglichkeit verbessern.

Unter Derivaten eines erfindungsgemäßen Proteins können im weitesten Sinne auch Präparationen dieser Proteine verstanden werden. Je nach Gewinnung, Aufarbeitung oder Präparation kann ein Protein mit diversen anderen Stoffen vergesellschaftet sein, beispielsweise aus der Kultur der produzierenden Mikroorganismen. Ein Protein kann auch, beispielsweise zur Erhöhung seiner Lagerstabilität, mit anderen Stoffen gezielt versetzt worden sein. Erfindungsgemäß sind deshalb auch alle Präparationen eines erfindungsgemäßen Proteins. Das ist auch unabhängig davon, ob es in einer bestimmten Präparation tatsächlich diese enzymatische Aktivität entfaltet oder nicht. Denn es kann gewünscht sein, daß es bei der Lagerung keine oder nur geringe Aktivität besitzt, und erst zum Zeitpunkt der Verwendung seine enzymatische Funktion entfaltet. Dies kann beispielsweise über entsprechende Begleitstoffe gesteuert werden.

Die erfindungsgemäßen Zusammensetzungen enthalten bevorzugt zusätzlich mindestens eine Lipase. Eine in der erfindungsgemäßen Zusammensetzung (insbesondere in einem erfindungsgemäß bevorzugten Wasch- und Reinigungsmittel für Textilien) enthaltene Lipase weist eine lipolytische Aktivität auf, das heißt, sie ist zur Hydrolyse (Lipolyse) von Lipiden wie Glyceriden oder Cholesterinestern befähigt. Diese Lipaseaktivität wird in fachüblicher Weise bestimmt, und zwar vorzugsweise wie beschrieben in Bruno Stellmach, "Bestimmungsmethoden Enzyme für Pharmazie, Lebensmittelchemie, Technik, Biochemie, Biologie, Medizin" (Steinkopff Verlag Darmstadt, 1988, S. 172 ff). Hierbei werden Lipase-haltige Proben zu einer Olivenölemulsion in emulgatorhaltigem Wasser gegeben und bei 30 °C und pH 9,0 inkubiert. Dabei werden Fettsäuren freigesetzt. Diese werden mit einem Autotitrator über 20 min. laufend mit 0,01 N Natronlauge titriert, so dass der pH-Wert konstant bleibt ("pH-stat-Titration"). Anhand des Natronlauge-Verbrauchs erfolgt mittels Bezug auf eine Referenzlipaseprobe die Bestimmung der Lipaseaktivität. Eine weitere geeignete Methode zur Messung der Lipaseaktivität ist die Freisetzung eines Farbstoff aus einem geeigneten pNP-gelabelten Substrat.

Bevorzugte Zusammensetzungen sind dadurch gekennzeichnet, dass bezogen auf das Gesamtgewicht der Zusammensetzung Lipase in einer Gesamtmenge von 0,01 bis 1,0 Gew.-%, insbesondere von 0,02 bis 0,1 Gew.-%, enthalten ist.

Erfindungsgemäß bevorzugte Lipase-Enzyme werden ausgewählt aus mindestens einem Enzym der Gruppe, die gebildet wird aus Triacylglycerol-Lipase (E.C. 3.1.1.3) und Lipoprotein-Lipase (E.C. 3.1.1.34) und Monoglycerid-Lipase (E.C. 3.1.1.23).

Das erfindungsgemäß bevorzugte Einsatzgebiet der erfindungsgemäßen Zusammensetzungen ist die Reinigung von Textilien. Weil Wasch- und Reinigungsmittel für Textilien überwiegend alkalische pH-Werte aufweisen, werden hierfür insbesondere Lipasen eingesetzt, die im alkalischen Medium aktiv sind.

Ferner ist die in einer erfindungsgemäßen Zusammensetzung bevorzugt enthaltene Lipase natürlicherweise in einem Mikroorganismus der Art *Thermomyces lanuginosus* oder *Rhizopus oryzae* oder *Mucor javanicus* vorhanden oder von vorgenannten natürlicherweise vorhandenen Lipasen per Mutagenese abgeleitet. Besonders bevorzugt enthalten die erfindungsgemäßen Zusammensetzungen mindestens eine Lipase, die natürlicherweise in einem Mikroorganismus der Art *Thermomyces lanuginosus* vorhanden oder sich von vorgenannten natürlicherweise in *Thermomyces lanuginosus* vorhandenen Lipasen per Mutagenese ableitet.

Natürlicherweise vorhanden bedeutet in diesem Zusammenhang, dass die Lipase ein eigenes Enzym des Mikroorganismus ist. Die Lipase kann folglich in dem Mikroorganismus von einer Nukleinsäuresequenz exprimiert werden, die Teil der chromosomalen DNA des Mikroorganismus in seiner Wildtyp-Form ist. Sie bzw. die für sie codierende Nukleinsäuresequenz ist folglich in der Wildtyp-Form des Mikroorganismus vorhanden und/oder kann aus der Wildtyp-Form des Mikroorganismus aus diesem isoliert werden. Im Gegensatz hierzu wäre eine nicht natürlicherweise in dem Mikroorganismus vorhandene Lipase bzw. die für sie codierende Nukleinsäuresequenz mit Hilfe gentechnischer Verfahren in den Mikroorganismus gezielt eingebracht worden, so dass der Mikroorganismus um die Lipase bzw. die für sie codierende Nukleinsäuresequenz bereichert worden wäre. Jedoch kann eine Lipase, die natürlicherweise in einem Mikroorganismus der Art *Thermomyces lanuginosus* oder *Rhizopus oryzae* oder *Mucor javanicus* vorhanden ist, aber durchaus rekombinant von einem anderen Organismus hergestellt worden sein.

Der Pilz *Thermomyces lanuginosus* (auch bekannt unter *Humicola lanuginosa*) zählt zur Klasse der Eurotiomycetes (Unterklasse Eurotiomycetidae), hierin zur Ordnung der Eurotiales und hierin zur Familie Trichocomaceae und der Gattung Thermomyces. Der Pilz *Rhizopus oryzae* zählt zur Klasse der Zygomyceten (Unterklasse Incertae sedis), hierin zur Ordnung Mucorales und hierin wiederum zur Familie Mucoraceae und der Gattung Rhizopus. Der Pilz *Mucor javanicus* zählt ebenfalls zur Klasse der Zygomyceten (Unterklasse Incertae sedis), hierin zur Ordnung Mucorales und hierin wiederum zur Familie Mucoraceae, hierin dann zur Gattung Mucor. Die Bezeichnungen *Thermomyces lanuginosus,* Rhizopus oryzae und Mucorjavanicus sind die biologischen Artbezeichnungen innerhalb der jeweiligen Gattung.

Erfindungsgemäß bevorzugte Lipasen sind die von dem Unternehmen Amano Pharmaceuticals unter den Bezeichnungen Lipase M-AP10^{®}, Lipase LE^{®} und Lipase F^{®} (auch Lipase JV^{®}) erhältlichen Lipaseenzyme. Die Lipase F^{®} ist beispielsweise natürlicherweise in Rhizopus oryzae vorhanden. Die Lipase M-AP10^{®} ist beispielsweise natürlicherweise in Mucor javanicus vorhanden

Zusammensetzungen einer ganz besonders bevorzugten Ausführungsform der Erfindung enthalten mindestens eine Lipase, die ausgewählt wird aus mindestens einem oder mehreren Polypeptiden mit einer Aminosäuresequenz, die zu mindestens 90 % (und zunehmend bevorzugt zu mindestens 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0 %, 99,1%, 99,2%, 99,3%, 99,4%, 99,5%, 99,6%, 99,7%, 99,8%, 99,9%) zur Wildtyp Lipase aus dem Stamm DSM 4109 *Thermomyces lanuginosus* identisch ist. Dabei ist es erneut bevorzugt, wenn ausgehend von besagter Wildtyp Lipase aus dem Stamm DSM 4109 zumindest die Aminosäureänderung N233R vorliegt.

Es sind im Rahmen einer weiteren Ausführungsform insbesondere solche Lipasen abgeleitet von der Wildtyp Lipase aus dem Stamm DSM 4109 erfindungsgemäß bevorzugt verwendbar, die ausgewählt werden aus mindestens einem Lipase-Enzym gemäß mindestens einem der Ansprüche 1 bis 13 der Druckschrift WO 00/60063 A1. Auf die Offenbarung Druckschrift WO 00/60063 A1 wird ausdrücklich vollumfänglich Bezug genommen.

Besonders bevorzugt wird in den Zusammensetzungen der Erfindung mindestens eine Lipase eingesetzt, die abgeleitet ist von der Wildtyp Lipase aus dem Stamm DSM 4109 und in der ausgehend von besagter Wildtyp Lipase mindestens eine Substitution einer elektrisch neutralen oder negativ geladenen Aminosäure durch eine positiv geladene Aminosäure erfolgte. Die Ladung wird in Wasser bei pH 10 bestimmt. Negative Aminosäuren im Sinne der Erfindung sind E, D, Y und C. Positiv geladene Aminosäuren im Sinne der Erfindung sind R, K und H, insbesondere R und K. Neutrale Aminosäure im Sinne der Erfindung sind G, A, V, L, I, P, F, W, S, T, M, N, Q und C, wenn C eine Disulfidbrücke ausbildet.

Im Rahmen dieser Ausführungsform der Erfindung ist es erneut bevorzugt, wenn ausgehend von der Wildtyp Lipase aus dem Stamm DSM 4109 mindestens einen der folgenden Aminosäureaustausche in den Positionen D96L, T213R und/oder N233R, besonders bevorzugt T213R und N233R, vorliegt.

Es hat sich herausgestellt, dass als ganz besonders bevorzugte Lipase in den erfindungsgemäßen Zusammensetzungen mindestens eine Lipase enthalten ist, die ausgehend von besagter Wildtyp Lipase aus dem Stamm DSM 4109 *Thermomyces lanuginosus* eine der folgenden

Aminosäureänderungen der Nummern (L1) bis (L41) als einzige Änderung aufweist (Änderungen in Klammern optional):

| | |
|---|---|
| (L1) | T231R+ N233R |
| (L2) | N94K+ D96L+ T231R+ N233R+ Q249R+ 270P+ 271G+ 272L |
| (L3) | D96L+ T231 R+ N233R |
| (L4) | G91A+ E99K+ T231R+ N233R+ Q249R |
| (L5) | (N33Q) +D96L + T231R +N233R +Q249R +270 PGL |
| (L6) | R209P +T231R +N233R |
| (L7) | (N33Q) +E99N +N101S +T231R +N233R +Q249R +270 PGL |
| (L8) | K24C +(N33Q) +D96S +T231R +N233R +Q249R +270 PCL |
| (L9) | (N33Q) +G91A +E99K +T231R +N233R +Q249R +270 PGL |
| (L10) | E1A +(N33Q) +G91A +E99K +T231 R +N233R +Q249R +270 PGL |
| (L11) | (N33Q) +G91A +E99K +G255R +T231 R +N233R +Q249R +270 PGL |
| (L12) | (N33Q) +G91A +E99K +T231 R +N233R +T244R +Q249R +270 PGL |
| (L13) | G91A +E99K +T231R +N233R +Q249R |
| (L14) | E87K +G91D +D96L +G225P +T231R +N233R +Q249R +N251D |
| (L15) | G91A +E99K +T231R +N233R +Q249R +270AGVF |
| (L16) | G91A +E99K +T189G +T231R +N233R +Q249R |
| (L17) | D102G +T231R +N233R +Q249R |
| (L18) | T231R +N233R +Q249R +270AGVF |
| (L19) | R209P +T231R +N233R |
| (L20) | N33Q +N94K +D96L +T231R +N233R +Q249R +270PGLPFKRV |
| (L21) | N33Q +N94K +D96L +T231R +N233R +Q249R |
| (L22) | N33Q +D96S +T231R +N233R +Q249R |
| (L23) | N33Q +D96S +V228I +T231R +N233R +Q249R |
| (L24) | E1A +N33Q +G91A +E99K +T231R +N233R +Q249R +270PGLPFKRV |
| (L25) | N33Q +S83T +E87K +G91A +E99K +T231R + N233R +Q249R +270PGLPFKRV |
| (L26) | N33Q +G91A +E99K +T231R +N233R +Q249R +270PGLPFKRV |
| (L27) | T231R +N233R +270CP |
| (L28) | T231R +N233R +270RE |
| (L29) | N33Q +E99N +N101S +T231R +N233R +Q249R +270PGLPFKRV |
| (L30) | D62A +S83T +G91A +E99K +T231R +N233R +Q249R |
| (L31) | E99N +N101S +T231R +N233R + Q249R |
| (L32) | R84W +G91A +E99K +T231R +N233R +Q249R |
| (L33) | G91A +E99K +T231R +N233R +Q249R +270SPG |
| (L34) | G91A +E99K +T231R +N233R +Q249R +270VVVP |
| (L35) | G91A +E99K +T231R +N233R +Q249R +270LLA88GRGGHR |
| (L36) | G91 A +E99K +T231R +N233R +Q249R +270VTT |
| (L37) | G91A +E99K +T231R +N233R +Q249R +270VLQ |
| (L38) | G91A +E99K +T231R +N233R +Q249R +270T8T |
| (L39) | G91A +E99K +T231R +N233R +Q249R +270LRI |
| (L40) | V60G +D62E +G91A +E99K +T231R +N233R +Q249R |
| (L41) | G91A +D96W +E99K +T231R +N233R +G263Q +L264A +1265T +G2668 +T267A +L269N +270AGGF8 |

Bevorzugte Zusammensetzungen dieser Ausführungsform sind dadurch gekennzeichnet, dass bezogen auf das Gesamtgewicht der Zusammensetzung besagtes Polypeptid in einer Gesamtmenge von 0,01 bis 1,0 Gew.-%, insbesondere von 0,02 bis 0,1 Gew.-%, enthalten ist.

Eine höchst bevorzugte Lipase ist kommerziell unter dem Handelsnamen Lipex^{®} von dem Unternehmen Novozymes (Dänemark) zu beziehen und vorteilhaft in den erfindungsgemäßen Reinigungszusammensetzungen einsetzbar. Besonders bevorzugt ist hierbei die Lipase Lipex^{®} 100 L (ex Novozymes A/S, Dänemark). Bevorzugte Zusammensetzungen sind dadurch gekennzeichnet, dass bezogen auf das Gesamtgewicht der Zusammensetzung besagtes Lipase-Enzym aus Lipex^{®} 100 L in einer Gesamtmenge von 0,01 bis 1,0 Gew.-%, insbesondere von 0,02 bis 0,1 Gew.-%, enthalten ist.

Die erfindungsgemäßen Zusammensetzungen enthalten bevorzugt zusätzlich mindestens eine Mannanase. Eine in der erfindungsgemäßen Zusammensetzung (insbesondere in einem erfindungsgemäß bevorzugten Wasch- und Reinigungsmittel für Textilien) enthaltene Mannanase katalysiert im Rahmen ihrer Mannanase-Aktivität die Hydrolyse von 1,4-beta-D-mannosidischen Bindungen in Mannanen, Galactomannanen, Glucomannanen und Galactoglucomannanen. Besagte erfindungsgemäße Mannanase-Enzyme werden gemäß Enzym Nomenklatur als E.C. 3.2.1.78 klassifiziert.

Die Mannanase-Aktivität eines Polypeptids bzw. Enzyms kann gemäß literaturbekannten Testmethoden bestimmt werden. Dabei wird beispielsweise eine Testlösung in Löcher mit 4 mm Durchmesser einer Agarplatte, enthaltend 0.2 Gew.-% AZGL Galactomannan (carob), i.e. Substrat für das endo-1,4-beta-D-Mannanase Essay, erhältlich unter Katalognummer I-AZGMA der Firma Megazyme (http://www.megazyme.com), eingebracht.

Geeignete erfindungsgemäße Zusammensetzungen enthalten beispielsweise die Mannanase, die unter dem Namen Mannaway^{®} von der Firma Novozymes vermarktet wird.

Mannanase-Enzyme wurden in zahlreichen *Bacillus* Organismen identifiziert:
WO 99/64619 offenbart Beispiele für flüssige, proteasehaltige Waschmittelzusammensetzungen mit hohem Gesamttensidgehalt von mindestens 20 Gew.-%, die zusätzlich Mannanase-Enzym umfassen.

Bevorzugterweise enthalten die erfindungsgemäßen Zusammensetzungen bezogen auf das Gesamtgewicht der Zusammensetzung Mannanase in einer Gesamtmenge von 0,01 bis 1,0 Gew.-%, insbesondere von 0,02 bis 0,1 Gew.-%.

Mannanase-Polypeptide aus Stämmen der *Thermoanaerobacter Gruppe,* wie *Caldicellulosiruptor, sind erfindungsgemäß bevorzugt geeignet.* Gleichfalls im Rahmen der Erfindung einsetzbar sind Mannanase-Polypeptide der Pilze *Humicola* oder *Scytalidium,* insbesondere der Species *Humicola insolens* oder *Scytalidium thermophilum.*

Es ist erfindungsgemäß besonders bevorzugt, wenn die erfindungsgemäßen Zusammensetzungen als Mannanase-Enzym mindestens ein Mannanase-Polypeptid aus gram-positiven alkalophilen Stämmen von *Bacillus,* insbesondere ausgewählt aus mindestens einem Vertreter der Gruppe aus *Bacillus subtilis, Bacillus lentus, Bacillus clausii, Bacillus agaradhaerens, Bacillus brevis, Bacillus stearothermophilus, Bacillus alkalophilus, Bacillus amyloliquefaciens, Bacillus coagulans, Bacillus circulans, Bacillus lautus, Bacillus thuringiensis, Bacillus cheniformis, and Bacillus sp., besonders bevorzugt ausgewählt aus mindestens einem Vertreter der Gruppe aus Bacillus sp. 1633, Bacillus sp. AA112, Bacillus clausii, Bacillus agaradhaerens and Bacillus licheniformis.*

Eine bevorzugte erfindungsgemäße Mannanase wird ausgewählt aus mindestens einem Vertreter aus der Gruppe, die gebildet wird aus
i) Polypeptiden, die eine Aminosäuresequenz umfassen, die mindestens 90% (zunehmend bevorzugt mindestens 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0 %, 99,1%, 99,2%, 99,3%, 99,4%, 99,5%, 99,6%, 99,7% oder 99,8%) Sequenzidentität zu dem Polypeptid gemäß SEQ ID No.1 (vgl. Sequenzprotokoll), und
ii) Polypeptiden, die ein Fragment von (i) sind.

Dabei ist es wiederum bevorzugt, wenn besagte bevorzugte Mannanase in einer Gesamtmenge von 0,01 bis 1,0 Gew.-%, insbesondere von 0,02 bis 0,1 Gew.-%, jeweils bezogen auf das Gesamtgewicht der Zusammensetzung, in der erfindungsgemäßen Zusammensetzung enthalten ist.

Ein bevorzugtes Mannanase-Enzym wird gemäß Anspruch 1 der WO 99/64619 offenbart, in der Beschreibung dieser WO-Druckschrift näher beschrieben und ist demnach ausgewählt aus mindestens einem Mannanase-Enzym, dass ausgewählt wird aus mindestens einem Vertreter aus der Gruppe, die gebildet wird aus
i) Polypeptiden, die durch den für das Mannanaseenzym kodierenden Teil der DNA-Sequenz kodiert werden können, die in das in Escherichia coli DSM 12197 vorliegende Plasmid kloniert ist,
ii) Polypeptiden, die eine Aminosäuresequenz umfassen, wie an den Positionen 33-340 von SEQ ID NO: 2 in WO 99/64619 gezeigt,
iii) Polypeptiden, die eine Aminosäuresequenz umfassen, wie an den Positionen 31-990 oder den Positionen 91-1470, jeweils von SEQ ID NO: 2 in WO 99/64619 gezeigt, oder
iv) Analoga der in (i) oder (ii) definierten Polypeptide, die mindestens 90% (und zunehmend bevorzugt zu mindestens 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0 %, 99,1%, 99,2%, 99,3%, 99,4%, 99,5%, 99,6%, 99,7%, 99,8%, 99,9%) Sequenzidentität zu dem Polypeptid haben, oder ein Fragment von (i), (ii) oder (iii) sind.

Zur Auslegung der Merkmale der zuvor definierten, erfindungsgemäß bevorzugten Mannanase-Enzyme (*vide supra*) ist ausdrücklich auch die Gesamtoffenbarung der WO 99/64619 vollumfänglich heranzuziehen. Ebenso gelten die in der WO 99/64619 als bevorzugt genannten Mannanase-Enzyme im Sinne der erfindungsgemäßen Zusammensetzung als bevorzugt.

Erfindungsgemäß bevorzugte Zusammensetzungen enthalten zusätzlich mindestens eine Protease. Eine Protease ist ein Enzym, das Peptidbindungen mittels Hydrolyse spaltet. Jedes der Enzyme aus der Klasse E.C. 3.4 fällt erfindungsgemäß darunter (umfassend jede der darunterfallenden dreizehn Unterklassen). Die EC-Nummer entspricht der Enzyme Nomenklatur 1992 der NC-IUBMB, Academic Press, San Diego, California, eingeschlossen der Ergänzungen 1 bis 5, publiziert in Eur. J. Biochem. 1994, 223, 1-5; Eur. J. Biochem. 1995, 232, 1-6; Eur. J. Biochem. 1996, 237, 1-5; Eur. J. Biochem. 1997, 250, 1-6; and Eur. J. Biochem. 1999, 264, 610-650.

Subtilase benennt eine Untergruppe der Serinproteasen. Die Serinproteasen oder Serinpeptidasen sind eine Untergruppe der Proteasen, die Serin im aktiven Zentrums des Enzyms besitzen, das ein kovalentes Addukt mit dem Substrat bildet. Weiterhin sind die Subtilasen (und die Serineproteasen) dadurch charakterisiert, dass sie neben besagtem Serin mit Histidin und Aspartam zwei weitere Aminosäurereste im aktiven Zentrum aufweisen. Die Subtilasen können in 6 Unterklassen, nämlich die Subtilisin Familie, die Thermitase Familie, die Proteinase K Familie, die Familie der lantibiotischen Peptidasen, die Kexin Familie und die Pyrolysin Familie. Die als Bestandteil der erfindungsgemäßen Zusammensetzungen bevorzugt ausgenommenen oder bevorzugt in reduzierten Mengen enthaltenen Proteasen sind Endopeptidasen (EC 3.4.21).

"Proteaseaktivität" liegt erfindungsgemäß vor, wenn das Enzym proteolytische Aktivität besitzt (EC 3.4). Verschiedenartige Proteaseaktivitäts-Typen sind bekannt: Die drei Haupttypen sind:
Trypsin-artig, wobei eine Spaltung des Amidesubstrates nach den Aminosäuren Arg oder Lys bei P1 erfolgt; Chymotrypsin-artig, wobei eine Spaltung nach einer der hydrophoben Aminosäuren bei P1 erfolgt; und Elastase-artig, wobei eine Spaltung des Amidsubstrates nach Ala bei P1 erfolgt.

Die Proteaseaktivität kann nach der in Tenside, Band 7 (1970), S. 125-132 beschriebenen Methode ermittelt werden. Sie wird dementsprechend in PE (Protease-Einheiten) angegeben. Die Proteaseaktivität eines Enzyms lässt sich gemäß gängigen Standardmethoden, wie insbesondere unter Einsatz von BSA als Substrat (Rinderalbumin) und/oder mit der AAPF-Methode.

Es ist erfindungsgemäß bevorzugt, wenn die flüssigen Zusammensetzungen zusätzlich mindestens eine Cellulase enthalten. Eine Cellulase ist ein Enzym. Für Cellulasen können synonyme Begriffe verwendet werden, insbesondere Endoglucanase, Endo-1,4-beta-Glucanase, Carboxymethylcellulase, Endo-1,4-beta-D-Glucanase, beta-1,4-Glucanase, beta-1,4-Endoglucanhydrolase, Celludextrinase oder Avicelase. Entscheidend dafür, ob ein Enzym eine Cellulase im Sinne der Erfindung ist, ist deren Fähigkeit zur Hydrolyse von 1,4-ß-D-glucosidischen Bindungen in Cellulose.

Erfindungsgemäß konfektionierbare Cellulasen (Endoglucanasen, EG) umfassen beispielsweise die pilzliche, Endoglucanase(EG)-reiche Cellulase-Präparation beziehungsweise deren Weiterentwicklungen, die von dem Unternehmen Novozymes unter dem Handelsnamen Celluzyme^{®} angeboten wird. Die ebenfalls von dem Unternehmen Novozymes erhältlichen Produkte Endolase^{®} und Carezyme^{®} basieren auf der 50 kD-EG, beziehungsweise der 43 kD-EG aus Humicola insolens DSM 1800. Weitere einsetzbare Handelsprodukte dieses Unternehmens sind Cellusoft^{®}, Renozyme^{®} und Celluclean^{®}. Weiterhin einsetzbar sind beispielsweise Cellulasen, die von dem Unternehmen AB Enzymes, Finnland, unter den Handelsnamen Ecostone^{®} und Biotouch^{®} erhältlich sind, und die zumindest zum Teil auf der 20 kD-EG aus Melanocarpus basieren. Weitere Cellulasen von dem Unternehmen AB Enzymes sind Econase^{®} und Ecopulp^{®}. Weitere geeignete Cellulasen sind aus Bacillus sp. CBS 670.93 und CBS 669.93, wobei die aus Bacillus sp. CBS 670.93 von dem Unternehmen Danisco/Genencor unter dem Handelsnamen Puradax^{®} erhältlich ist. Weitere verwendbare Handelsprodukte des Unternehmens Danisco/Genencor sind "Genencor detergent cellulase L" und IndiAge^{®}Neutra. Auch durch Punktmutationen erhältliche Varianten dieser Enzyme können erfindungsgemäß eingesetzt werden. Besonders bevorzugte Cellulasen sind Thielavia terrestris Cellulasevarianten, die in der internationalen Offenlegungsschrift WO 98/12307 offenbart sind, Cellulasen aus Melanocarpus, insbesondere Melanocarpus albomyces, die in der internationalen Offenlegungsschrift WO 97/14804 offenbart sind, Cellulasen vom EGIII-Typ aus Trichoderma reesei, die in der europäischen Patentanmeldung EP 1 305 432 offenbart sind bzw. hieraus erhältliche Varianten, insbesondere diejenigen, die offenbart sind in den europäischen Patentanmeldungen EP 1240525 und EP 1305432, sowie Cellulasen, die offenbart sind in den internationalen Offenlegungsschriften WO 1992006165, WO 96/29397 und WO 02/099091. Auf deren jeweilige Offenbarung wird daher ausdrücklich verwiesen bzw. deren diesbezüglicher Offenbarungsgehalt wird daher ausdrücklich in die vorliegende Patentanmeldung mit einbezogen.

Erfindungsgemäß besonders bevorzugte Zusammensetzungen sind dadurch gekennzeichnet, dass als zusätzliche Cellulase mindestens eine Cellulase aus *Melanocarpus sp.* oder *Myriococcum sp.* erhältlicher 20K-Cellulase oder solcher, die eine Homologie von über 80 % (zunehmend bevorzugt von über 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0 %, 99,1%, 99,2%, 99,3%, 99,4%, 99,5%, 99,6%, 99,7%, 99,8%, 99,9%) dazu aufweist.

Die aus Melanocarpus sp. oder Myriococcum sp. erhältliche 20K-Cellulase ist aus der internationalen Patentanmeldung WO 97/14804 bekannt. Sie besitzt wie dort beschrieben ein Molekulargewicht von etwa 20 kDa und weist bei 50 °C im pH-Bereich von 4 bis 9 mindestens 80 % ihrer maximalen Aktivität auf, wobei noch fast 50 % der maximalen Aktivität bei pH 10 erhalten bleiben. Sie kann, wie ebenfalls dort beschrieben, aus Melanocarpus albomyces isoliert und in gentechnisch hergestellten Trichoderma reseei-Transformanten produziert werden. Im Sinne der vorliegenden Erfindung brauchbar sind auch Cellulasen, die eine Homologie von über 80 % (zunehmend bevorzugt von über 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0 %, 99,1%, 99,2%, 99,3%, 99,4%, 99,5%, 99,6%, 99,7%, 99,8%, 99,9%) zur 20K-Cellulase aufweisen.

K20-Cellulase wird vorzugsweise in solchen Mengen verwendet, daß eine erfindungsgemäße Zusammensetzung eine cellulolytische Aktivität von 1 NCU/g bis 500 NCU/g (bestimmbar durch die Hydrolyse von 1-gewichtsprozentiger Carboxymethylcellulose bei 50 °C und neutralem pH und Bestimmung der dabei freigesetzten reduzierenden Zucker mittels Dinitrosalicylsäure, wie von M.J.Bailey et al. in Enzyme Microb. Technol. 3: 153 (1981) beschrieben; 1 NCU definiert die Enzymmenge, die reduzierenden Zucker in einer Menge erzeugt, die 1 nmol Glukose pro Sekunde entspricht), insbesondere von 2 NCU/g bis 400 NCU/g und besonders bevorzugt von 6 NCU/g bis 200 NCU/g aufweist. Daneben kann die erfindungsgemäße Zusammensetzung gegebenenfalls noch weitere Cellulasen enthalten.

Eine erfindungsgemäße Zusammensetzung enthält vorzugsweise 0,001 mg bis 0,5 mg, insbesondere 0,02 mg bis 0,3 mg an cellulolytischem Protein pro Gramm der gesamten Zusammensetzung. Die Proteinkonzentration kann mit Hilfe bekannter Methoden, zum Beispiel dem Bicinchonsäure-Verfahren (BCA-Verfahren, Pierce Chemical Co., Rockford, IL) oder dem Biuret-Verfahren (A.G. Gornall, C.S. Bardawill und M.M. David, J. Biol. Chem. 177, 751-766, 1948) bestimmt werden.

Es ist erfindungsgemäß wiederum besonders bevorzugt, zusätzlich zu mindestens einer ersten Cellulase aus *Melanocarpus sp.* oder *Myriococcum sp.* erhältlicher 20K-Cellulase oder solcher, die eine Homologie von über 80 % % (zunehmend bevorzugt von über 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0 %, 99,1%, 99,2%, 99,3%, 99,4%, 99,5%, 99,6%, 99,7%, 99,8%, 99,9%) dazu aufweist mindestens eine weitere von der ersten Cellulase verschiedene zweite Cellulase einzusetzen.

Im allgemeinen können die in einer erfindungsgemäßen Zusammensetzung enthaltenen Enzyme an Trägerstoffe adsorbiert und/oder in Hüllsubstanzen eingebettet sein, um sie gegen vorzeitige Inaktivierung zu schützen.

Erfindungsgemäße Zusammensetzungen können die erhaltenen Enzyme in jeder nach dem Stand der Technik etablierten Form zugesetzt werden. Hierzu gehören insbesondere die durch Granulation, Extrusion oder Lyophilisierung erhaltenen festen Präparationen, vorteilhafterweise möglichst konzentriert, wasserarm und/oder mit Stabilisatoren versetzt. In einer alternativen Darreichungsform können die Enzyme auch verkapselt werden, beispielsweise durch Sprühtrocknung oder Extrusion der Enzymlösung zusammen mit einem, vorzugsweise natürlichen Polymer oder in Form von Kapseln, beispielsweise solchen, bei denen die Enzyme wie in einem erstarrten Gel eingeschlossen sind, oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Die erfindungsgemäße Zusammensetzung weist zwingend bei 25°C und 1013 mbar eine Wasseraktivität a_{w} von mehr als 0,3 bis maximal 0,9 auf.

Es ist erfindungsgemäß besonders bevorzugt, wenn die flüssige Zusammensetzung bei 25°C und 1013 mbar eine Wasseraktivität a_{w} von maximal 0,9, insbesondere von maximal 0,8, ganz besonders bevorzugt von maximal 0,7, am bevorzugtesten von maximal 0,6 aufweist. Dabei gilt für jedes vorgenannte bevorzugte Maximum, dass die flüssige Zusammensetzung bei 25°C und 1013 mbar eine Wasseraktivität a_{w} von mehr als 0,3, bevorzugt von mindestens 0,4, aufweist.

Insbesondere, wenn die erfindungsgemäße flüssige Zusammensetzung mit einer wasserlöslichen Folie umhüllt als Portion vorliegen soll, weist die erfindungsgemäße Zusammensetzung bevorzugt bei 25°C und 1013 mbar eine Wasseraktivität a_{w} zwischen 0,3 und 0,6, bevorzugt in einem Bereich von 0,35 bis 0,55, auf. Dabei ist es wiederum ganz besonders bevorzugt, wenn diese Zusammensetzungen Tensid als Tensidkombination eine der als bevorzugt gekennzeichneten Tensidkombinationen (vide supra), insbesondere eine der vorgenannten Tensidkombinationen (A) bis (D), enthalten ist.

Die Wasseraktivität a_{w} ist eine Zahl von 0 bis 1. Sie ist ein Maß für den Gehalt an freiem Wasser in der Zusammensetzung. Die Wasseraktivität lässt sich durch Messung der Luftfeuchtigkeit über der Probe in einer abgeschlossenen Probenkammer bei 25°C und 1013 mbar bestimmen. Dabei wird die Feuchtigkeit der Luft über der Probe im Gleichgewichtszustand in %RH ermittelt (relative Feuchtigkeit in %). Dabei werden %RH und Temperatur gemessen, bis sich ein Gleichgewicht für %RH und Temperatur (bei 25°C) einstellt.

Der Wert a_{w} steht in linearer Relation zur gemessenen relativen Feuchtigkeit %RH (1,00 a_{w} = 100% RH).

Das zur Bestimmung des a_{w}-Wertes verwendete Messgerät wird mittels standardisierter, gesättigter Salzlösungen gemäß National Bureau of Standards (NBS), Washington DC, (Journal of Research of the National Bureau of Standards, Volume 81A, No.1, Jan./Feb. 1977) und gemäß physikalisch-technischer Bundesanstalt, Berlin (Testreport Nr. PTB-2.54-1063/-4180/93, Aug. 1993) kalibriert.

Die Wasseraktivität a_{w} wurde mit dem Messinstrument LabMaster-aw (Sensorzell-Typ: CM-2) der Firma Novasina (Schweiz) bestimmt. Zur Kalibrierung der Messung der relativen Luftfeuchtigkeit wurden die standardisierten Salzlösungen SAL-T 6 (6 %RH), SAL-T 11 (11 %RH), SAL-T 33 (33 %RH), SAL-T 58 (58 %RH), SAL-T 75 (75 %RH), SAL-T 84 (84 %RH) und SAL-T 97 (97 %RH) (jeweils Firma Novasina) jeweils gemäß o.g. Standards verwendet. Die Kalibrierung des Geräts wurde gemäß vorgenannter Standards durch die Firma Novasina vorgenommen und zertifiziert.

Zur Bestimmung des a_{w}-Werts wurde die Messsubstanz (7,5 mL) in ein Probengefäß des vorgenannten Messinstrumentes gefüllt, das Probengefäß sofort in die Messkammer eingebracht und die Messkammer geschlossen. Die Messtemperatur in der Messkammer wurde auf 25°C geregelt. Wenn der a_{w}-Wert 8 Minuten lang stabil blieb (± 0.001 a_{w}) (Geräteeinstellung a_{w}-Faktor: 8 Minuten) und die Temperatur 4 Minuten lang stabil blieb (25°C ± 0.1°C) (Geräteeinstellung Temperaturfaktor: 4 Minuten) hatte sich der Gleichgewichtszustand eingestellt und der Messwert wurde als a_{w} ausgegeben.

Zusätzlich zu den zwingend enthaltenen Inhaltsstoffen können die erfindungsgemäßen Zusammensetzungen weitere Inhaltsstoffe enthalten, die die anwendungstechnischen und/oder ästhetischen Eigenschaften des Waschmittels weiter verbessern. Im Rahmen der vorliegenden Erfindung enthält die erfindungsgemäße Zusammensetzung vorzugsweise zusätzlich einen oder mehrere Stoffe aus der Gruppe der Bleichmittel, Komplexbildner, Gerüststoffe, Elektrolyte, pH-Stellmittel, Parfüme, Parfümträger, Fluoreszenzmittel, Farbstoffe, Hydrotrope, Schauminhibitoren, Silikonöle, Antiredepositionsmittel, Einlaufverhinderer, Knitterschutzmittel, Farbübertragungsinhibitoren, antimikrobiellen Wirkstoffe, Germizide, Fungizide, Antioxidantien, Konservierungsmittel, Korrosionsinhibitoren, Antistatika, Bittermittel, Bügelhilfsmittel, Phobier- und Imprägniermittel, Quell- und Schiebefestmittel, weichmachenden Komponenten sowie UV-Absorber.

Als Bleichmittel können alle Stoffe dienen, die durch Oxidation, Reduktion oder Adsorption Farbstoffe zerstören bzw. aufnehmen und dadurch Materialien entfärben. Dazu gehören unter anderem hypohalogenithaltige Bleichmittel, Wasserstoffperoxid, Perborat, Percarbonat, Peroxoessigsäure, Diperoxoazelainsäure, Diperoxododecandisäure und oxidative Enzymsysteme.

Als Gerüststoffe, die in der erfindungsgemäßen Zusammensetzung enthalten sein können, sind insbesondere Silikate, Aluminiumsilikate (insbesondere Zeolithe), Carbonate, Salze organischer Di- und Polycarbonsäuren sowie Mischungen dieser Stoffe zu nennen.

Organische Gerüststoffe, welche in der erfindungsgemäßen Zusammensetzung vorhanden sein können, sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Als Gerüststoffe sind weiter polymere Polycarboxylate geeignet. Dies sind beispielsweise die Alkalimetallsalze der Polyacrylsäure oder der Polymethacrylsäure, zum Beispiel solche mit einer relativen Molekülmasse von 600 bis 750.000 g / mol.

Geeignete Polymere sind insbesondere Polyacrylate, die bevorzugt eine Molekülmasse von 1.000 bis 15.000 g / mol aufweisen. Aufgrund ihrer überlegenen Löslichkeit können aus dieser Gruppe wiederum die kurzkettigen Polyacrylate, die Molmassen von 1.000 bis 10.000 g / mol, und besonders bevorzugt von 1.000 bis 5.000 g / mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Zur Verbesserung der Wasserlöslichkeit können die Polymere auch Allylsulfonsäuren, wie Allyloxybenzolsulfonsäure und Methallylsulfonsäure, als Monomer enthalten.

In den erfindungsgemäßen flüssigen Zusammensetzungen werden bevorzugt lösliche Gerüststoffe, wie beispielsweise Citronensäure, oder Acrylpolymere mit einer Molmasse von 1.000 bis 5.000 g / mol eingesetzt.

Ein zweiter Erfindungsgegenstand ist die Verwendung einer flüssigen Zusammensetzung des ersten Erfindungsgegenstandes zur Stabilisierung von α-Amylase, die zu der in SEQ ID NO.1 angegeben Sequenz über deren Gesamtlänge zu mindestens 89% und zunehmend bevorzugt zu mindestens 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99%, 99,5% und bis zu 100% identisch ist und in der Zählung gemäß SEQ ID NO. 1 an einer oder mehreren der Positionen 180, 181, 182, 183 und 184 Deletionen aufweist.

Ein dritter Erfindungsgegenstand ist die Verwendung einer flüssigen Zusammensetzung des ersten Erfindungsgegenstandes zur Reinigung von Textilien.

Ein vierter Gegenstand der Erfindung ist ein Verfahren zur Textilreinigung, umfassend die Bereitstellung einer Waschflotte unter Einsatz von mindestens folgenden Komponenten
(i) mindestens einer Zusammensetzung des ersten Erfindungsgegenstandes,
(ii) bevorzugt mindestens einem Lösemittel, insbesondere Wasser, und
(iii) mindestens einem Textil.

Eine Waschflotte ist erfindungsgemäß zumindest die Gesamtmenge der unter (i) und (iii) aufgezählten Komponenten.

Verfahren zur Textilreinigung zeichnen sich im allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einer Zusammensetzung des ersten Erfindungsgegenstandes oder einer Lösung dieser Zusammensetzung behandelt wird.

Wird der Waschflotte zusätzlich Komponente (ii) des erfindungsgemäßen Verfahrens zugeführt, so ist es erfindungsgemäß bevorzugt, einen Volumenteil der Komponente (i) mit 5 bis 3000 Volumenteilen der Komponente (ii) zu kombinieren.

In den beschriebenen Verfahren werden in verschiedenen Ausführungsformen der Erfindung Temperaturen von 60 °C oder weniger, 40 °C oder weniger, 30°C oder weniger oder 20°C oder weniger, eingesetzt. Diese Temperaturangaben beziehen sich auf die in den Waschschritten eingesetzten Temperaturen.

### Beispiele

Folgende Zusammensetzungen wurden hergestellt:

### 1. Flüssigwaschmittel (nicht erfindungsgemäß):

| Inhaltsstoff | V1 Menge in Gew.-% | E1 Menge in Gew.-% |
|---|---|---|
| Borsäure | 1,0 | 1,0 |
| Zitronensäure Monohydrat | 2,2 | 3,2 |
| Fettalkoholethersulfat mit 2 Mol EO | 7,0 | 9,0 |
| Fettalkoholether mit 7 EO | 5,5 | 9,0 |
| Alkylbenzolsulfonsäure | 5,5 | 7,0 |
| NaOH | 2,33 | 2,95 |
| Glyzerin | 2,5 | - |
| Diethylenetriaminpenta(methylenphosphonsäure), Heptanatriumsalz | 0,3 | 0,3 |
| Fettsäureseife | 2,2 | 4,0 |
| Ethanol | 1,2 | 2,0 |
| 1,2-Propandiol | - | 5,5 |
| Amylase (erfindungsgemäß) | 0,44 | 0,44 |
| Mannanase | 0,10 | 0,10 |
| Lipase | 0,20 | 0,20 |
| Cellulase | 0,15 | 0,15 |
| Protease | 0,65 | 0,65 |
| Pectatlyase | 0,05 | 0,05 |
| Wasser | Ad 100 | Ad 100 |
| Wasseraktivität a_{w} | 0.956 | 0,928 |

Es wurde die Wasseraktivität bestimmt (Messprotokoll *vide supra*).

Die Zusammensetzung E1 wies nach einer Lagerung von 4 Wochen bei 30°C eine höhere Amylaseaktivität auf als die Vergleichszusammensetzung V1.

| Formel | Anfangswert | Amylaseaktivität nach 2 Wochen Lagerung bei 40°C | Amylaseaktivität nach 4 Wochen Lagerung bei 40°C |
|---|---|---|---|
| E1 | 100% | 90 % | 60% |
| V1 | 100 % | 71 % | 47% |

### Ermittlung der Waschleistung im Waschtest

In einer Waschmaschine vom Typ Miele Softtronic W 1734 wurde im Waschprogramm "easy care" standardisierte weiße Testwäsche (3,5 kg) in Gegenwart von jeweils frisch standardisiert beschmutzter Wäsche für einen Zeitraum von 70 Minuten und einem Wasserlevel von 17 Litern (Härte 16 d) bei 30°C gewaschen.

Die Zusammensetzung E1 wies die beste Waschleistung auf.

### 2.0 Waschmittel-Pouch mit fester und flüssiger Zusammensetzung

Es wurden folgende Zusammensetzungen hergestellt und wie nachfolgend beschrieben in einer wasserlöslichen Folie aus Polyvinylalkohol verpackt:

| Flüssige Zusammensetzung | L1 [Gew.-%] |
|---|---|
| C₁₁₋₁₃-Alkylbenzolsulfonsäure | 23,0 |
| C₁₃₋₁₅-Alkylalkohol ethoxyliert mit 8 Mol Ethylenoxid | 24,0 |
| Glyzerin | 9,0 |
| 2-Aminoethanol | 6,8 |
| ethoxyliertes Polyethylenimin | 4,0 |
| C₁₂₋₁₈-Fettsäure | 7,5 |
| Diethylentriamin-N,N,N',N',N"-penta(methylenphosphonsäure), Heptanatriumsalz | 3,5 |
| 1,2-Propylenglykol | 4,5 |
| Ethanol | 4,0 |
| Soil-Release Polymer aus Ethylenterephthalat und Polyethylenoxid-terephthalat | 1,0 |
| Amylase (erfindungsgemäß) | 0,6 |
| Parfüm, Farbstoff | 1,7 |
| Wasser | ad 100 |
| Wasseraktivität a_{w} | 0,42 |

| Festförmige Zusammensetzung (Granulat) | F1 [Gew.-%] |
|---|---|
| Natriumpercarbonat (Granulat) | 35,0 |
| TAED | 13,0 |
| Carboxymethylcellulose | 4,0 |
| Natriumhydrogencarbonat | 9,0 |
| Natriumsilikat (SiO₂)_{2.5}(Na₂O) | 11,0 |
| C18-Fettsäureseife | 2,6 |
| Enzyme (Protease, Lipase, Mannanase) im Granulat aus Natriumsulfat | 10,7 |
| Natriumsulfat | ad 100 |

Eine Folie M8613 der Fa. Monosol (88 µm) wurde auf eine Form mit Doppelkavität aufgespannt. Die aufgespannte Folie wurde für eine Dauer von 2400 ms bei 105°C durch Kontaktheizung erhitzt und dann durch ein Vakuum in die Kavität gezogen. Im Anschluss wurden 8,5 g der festförmigen Zusammensetzung F1 vorgewogen in die erste Kavität gefüllt und danach 16,5 g der flüssigen Zusammensetzung L1 mittels einer Spritze in die zweite Kavität gegeben. Danach wurde eine Oberfolie (M8630, 90 µm) zum Verschließen der Kavitäten aufgelegt und mittels Hitze (150°C, 1000 ms) mit der ersten Folie verschweißt. Nach dem Brechen des Vakuums wurde die Portion der Kavität entnommen. Eine Wandung der Pulverkammer der Portion wurde im Anschluss mit einer Nadel perforiert.

## Patentansprüche

1. Flüssige Zusammensetzung, insbesondere für die Reinigung von Textilien, enthaltend
(a) mindestens ein Tensid, und
(b) mindestens eine α-Amylase, die zu der in SEQ ID NO.1 angegeben Sequenz über deren Gesamtlänge zu mindestens 89% und zunehmend bevorzugt zu mindestens 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99%, 99,5% und bis zu 100% identisch ist und in der Zählung gemäß SEQ ID NO. 1 an einer oder mehreren der Positionen 180, 181, 182, 183 und 184 Deletionen aufweist,
mit der Maßgabe, dass die Zusammensetzung bei 25°C und 1013 mbar eine Wasseraktivität a_{w} von mehr als 0,3 bis maximal 0,9 aufweist,
**dadurch gekennzeichnet, dass** der Gehalt an Wasser weniger als 30 Gew.-% beträgt.

2. Flüssige Zusammensetzung nach Anspruch 1, **dadurch gekennzeichnet, dass** die Zusammensetzung bei 25°C und 1013 mbar eine Wasseraktivität a_{w} von maximal 0,8, besonders bevorzugt von maximal 0,7, am bevorzugtesten von maximal 0,6, aufweist.

3. Flüssige Zusammensetzung nach Anspruch 2, **dadurch gekennzeichnet, dass** die Zusammensetzung bei 25°C und 1013 mbar eine Wasseraktivität a_{w} von mindestens 0,4, aufweist.

4. Flüssige Zusammensetzung nach einem der Ansprüche 1 bis 3, **dadurch gekennzeichnet, dass** auf das Gesamtgewicht der Zusammensetzung bezogen Tensid in einer Gesamtmenge von 5,0 bis 60,0 Gew.-%, bevorzugt von 10,0 bis 40,0 Gew.-%, besonders bevorzugt von 15,0 bis 36,0 Gew.-%, enthalten ist.

5. Flüssige Zusammensetzung nach einem der Ansprüche 1 bis 4, **dadurch gekennzeichnet, dass** als anionisches Tensid mindestens ein Tensid der Formel (A-3) enthalten, 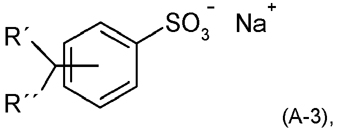 in der R' und R" zusammen 9 bis 19, vorzugsweise 11 bis 15 und insbesondere 11 bis 13 C-Atome enthalten,

6. Flüssige Zusammensetzung nach einem der Ansprüche 1 bis 5, **dadurch gekennzeichnet, dass** als anionisches Tensid mindestens ein Fettalkoholethersulfat der Formel A-1 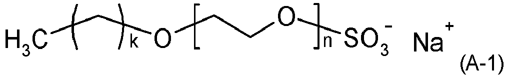 mit k = 11 bis 19, n = 2, 3, 4, 5, 6, 7 oder 8, enthalten ist.

7. Flüssige Zusammensetzung nach einem der Ansprüche 1 bis 6, **dadurch gekennzeichnet, dass** als Tensid mindestens ein nichtionisches Tensid enthalten ist.

8. Flüssige Zusammensetzung nach einem der Ansprüche 1 bis 7, **dadurch gekennzeichnet, dass** die Amylase Deletionen an mindestens zwei Positionen ausgewählt aus den Positionen 180+181, 181+182, 182+183 und 183+184 in der Zählung gemäß SEQ ID NO. 1, und ganz besonders bevorzugt an den Positionen 183+184 in der Zählung gemäß SEQ ID NO. 1 aufweist.

9. Flüssige Zusammensetzung nach einem der Ansprüche 1 bis 8, **dadurch gekennzeichnet, dass** die Amylase die Deletionen H183* + G184* in der Zählung gemäß SEQ ID NO. 1 aufweist.

10. Flüssige Zusammensetzung nach einem der Ansprüche 1 bis 9, **dadurch gekennzeichnet, dass** die Amylase in der Zählung gemäß SEQ ID NO. 1 weiterhin Aminosäuresubstitutionen an einer oder mehreren der Positionen 405, 421, 422 und 428 aufweist.

11. Flüssige Zusammensetzung nach einem der Ansprüche 1 bis 10, **dadurch gekennzeichnet, dass** die Amylase in der Zählung gemäß SEQ ID NO. 1 die Substitutionen I405L; A421H, A422P und A428T aufweist.

12. Flüssige Zusammensetzung nach einem der Ansprüche 1 bis 11, **dadurch gekennzeichnet, dass** zusätzlich mindestens eine Protease enthalten ist.

13. Flüssige Zusammensetzung nach einem der Ansprüche 1 bis 12, **dadurch gekennzeichnet, dass** zusätzlich mindestens eine Lipase enthalten ist, ausgewählt aus einem oder mehreren Polypeptiden mit einer Aminosäuresequenz, die zu mindestens 90 % zur Wildtyp Lipase aus dem Stamm DSM 4109 *Thermomyces lanuginosus* identisch ist.

14. Flüssige Zusammensetzung nach Anspruch 13, **dadurch gekennzeichnet, dass** mindestens eine Lipase enthalten ist, ausgewählt aus einem oder mehreren Polypeptiden mit einer Aminosäuresequenz abgeleitet von der Wildtyp Lipase aus dem Stamm DSM 4109, bei der mindestens einer der folgenden Aminosäureaustausche in den Positionen D96L und/oder T213R und/oder N233R, bevorzugt mindestens T213R und N233R, vorliegt.

## Claims

1. Liquid composition, in particular for cleaning textiles, containing
(a) at least one surfactant, and
(b) at least one α-amylase which is at least 89% and increasingly preferably at least 90%, 90.5%, 91%, 91.5%, 92%, 92.5%, 93%, 93.5%, 94%, 94.5%, 95%, 95.5%, 96%, 96.5%, 97%, 97.5%,
98%, 98.5%, 99%, 99.5% and up to 100% identical to the sequence specified in SEQ ID NO.1 over its total length and has deletions at one or more of positions 180, 181, 182, 183 and 184 in the count according to SEQ ID NO. 1,
with the proviso that the composition has a water activity a_{w} of more than 0.3 to a maximum of 0.9 at 25°C and 1013 mbar,
**characterized in that** the water content is less than 30% by weight.

2. Liquid composition according to claim 1, **characterized in that** the composition has a water activity a_{w} at 25°C and 1013 mbar of at most 0.8, particularly preferably of at most 0.7, most preferably of at most 0.6.

3. Liquid composition according to claim 2, **characterized in that** the composition has a water activity a_{w} of at least 0.4 at 25°C and 1013 mbar.

4. Liquid composition according to one of claims 1 to 3, **characterized in that** surfactant is present in a total amount of 5.0 to 60.0% by weight, preferably 10.0 to 40.0% by weight, particularly preferably 15.0 to 36.0% by weight, based on the total weight of the composition.

5. Liquid composition according to any one of claims 1 to 4, **characterized in that** at least one surfactant of the formula (A-3) is present as anionic surfactant, 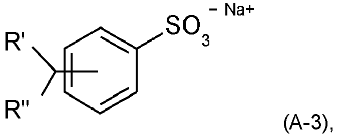 in which R' and R" together contain 9 to 19, preferably 11 to 15 and in particular 11 to 13 carbon atoms,

6. Liquid composition according to one of claims 1 to 5, **characterized in that** it contains at least one fatty alcohol ether sulfate of the formula A-1 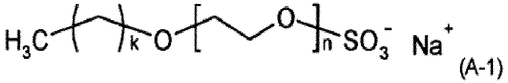 with k = 11 to 19, n = 2, 3, 4, 5, 6, 7 or 8 as anionic surfactant.

7. Liquid composition according to any one of claims 1 to 6, **characterized in that** at least one nonionic surfactant is present as surfactant.

8. Liquid composition according to any one of claims 1 to 7, **characterized in that** the amylase has deletions at at least two positions selected from positions 180+181, 181+182, 182+183 and 183+184 in the count according to SEQ ID NO. 1, and most preferably at positions 183+184 in the count according to SEQ ID NO. 1.

9. Liquid composition according to any one of claims 1 to 8, **characterized in that** the amylase has the deletions H183* + G184* in the count according to SEQ ID NO. 1.

10. Liquid composition according to any one of claims 1 to 9, **characterized in that** the amylase further comprises amino acid substitutions at one or more of positions 405, 421, 422 and 428 in the count according to SEQ ID NO. 1.

11. Liquid composition according to any one of claims 1 to 10, **characterized in that** the amylase has the substitutions I405L; A421H, A422P and A428T in the count according to SEQ ID NO. 1.

12. Liquid composition according to any one of claims 1 to 11, **characterized in that** at least one protease is additionally present.

13. Liquid composition according to any one of claims 1 to 12, **characterized in that** it additionally comprises at least one lipase selected from one or more polypeptides having an amino acid sequence which is at least 90% identical to the wild-type lipase from strain DSM 4109 *Thermomyces lanuginosus.*

14. Liquid composition according to claim 13, **characterized in that** at least one lipase is present, selected from one or more polypeptides having an amino acid sequence derived from the wild-type lipase from strain DSM 4109, in which at least one of the following amino acid substitutions is present in positions D96L and/or T213R and/or N233R, preferably at least T213R and N233R.

## Revendications

1. Composition liquide, en particulier pour le nettoyage des textiles, contenant
(a) au moins un agent tensioactif, et
(b) au moins une α-amylase qui correspond à la séquence indiquée dans SEQ ID NO.1 sur sa longueur totale à au moins 89% et de manière de plus en plus préférée à au moins 90%, 90,5%, 91%, 91,5%, 92%, 92,5%, 93%, 93,5%, 94%, 94,5%, 95%, 95,5%, 96%, 96,5%, 97%, 97,5%,
98%, 98,5%, 99%, 99,5% et jusqu'à 100% et présente des délétions dans le comptage selon SEQ ID NO. 1 à une ou plusieurs des positions 180, 181, 182, 183 et 184,
à condition que la composition présente une activité de l'eau a_{w} de plus de 0,3 à 0,9 au maximum à 25°C et 1013 mbar,
**caractérisé en ce que** la teneur en eau est inférieure à 30 % en poids.

2. Composition liquide selon la revendication 1, **caractérisée en ce que** la composition présente à 25°C et 1013 mbar une activité de l'eau a_{w} d'au maximum 0,8, de manière particulièrement préférée d'au maximum 0,7, de manière la plus préférée d'au maximum 0,6.

3. Composition liquide selon la revendication 2, **caractérisée en ce que** la composition présente une activité de l'eau a_{w} d'au moins 0,4, à 25°C et 1013 mbar.

4. Composition liquide selon l'une quelconque des revendications 1 à 3, **caractérisée en ce que**, par rapport au poids total de la composition, le tensioactif est présent en une quantité totale de 5,0 à 60,0 % en poids, de préférence de 10,0 à 40,0 % en poids, de manière particulièrement préférée de 15,0 à 36,0 % en poids.

5. Composition liquide selon l'une quelconque des revendications 1 à 4, **caractérisée en ce qu'**elle comprend, comme agent tensioactif anionique, au moins un agent tensioactif de formule (A-3), 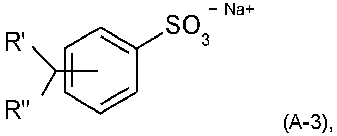 dans laquelle R' et R" contiennent ensemble de 9 à 19, de préférence de 11 à 15 et en particulier de 11 à 13 atomes de carbone,

6. Composition liquide selon l'une quelconque des revendications 1 à 5, **caractérisée en ce que**, comme tensioactif anionique, on utilise au moins un sulfate d'éther d'alcool gras de formule A-1 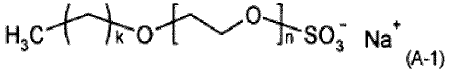 avec k = 11 à 19, n = 2, 3, 4, 5, 6, 7 ou 8.

7. Composition liquide selon l'une quelconque des revendications 1 à 6, **caractérisée en ce qu'**elle comprend comme agent tensioactif au moins un agent tensioactif non ionique.

8. Composition liquide selon l'une quelconque des revendications 1 à 7, **caractérisée en ce que** l'amylase présente des délétions en au moins deux positions choisies parmi les positions 180+181, 181+182, 182+183 et 183+184 dans le comptage selon la SEQ ID NO. 1, et de manière tout à fait préférée en positions 183+184 dans le comptage selon la SEQ ID NO. 1.

9. Composition liquide selon l'une quelconque des revendications 1 à 8, **caractérisée en ce que** l'amylase présente les délétions H183* + G184* dans le comptage selon SEQ ID NO. 1.

10. Composition liquide selon l'une quelconque des revendications 1 à 9, **caractérisée en ce que** l'amylase présente en outre des substitutions d'acides aminés à une ou plusieurs des positions 405, 421, 422 et 428 dans le comptage selon SEQ ID NO. 1.

11. Composition liquide selon l'une quelconque des revendications 1 à 10, **caractérisée en ce que** l'amylase présente les substitutions I405L ; A421H, A422P et A428T dans le comptage selon SEQ ID NO. 1.

12. Composition liquide selon l'une quelconque des revendications 1 à 11, **caractérisée en ce qu'**elle contient en outre au moins une protéase.

13. Composition liquide selon l'une quelconque des revendications 1 à 12, **caractérisée en ce qu'**elle contient en outre au moins une lipase choisie parmi un ou plusieurs polypeptides ayant une séquence d'acides aminés qui est identique à au moins 90 % à la lipase de type sauvage provenant de la souche DSM 4109 *Thermomyces lanuginosus.*

14. Composition liquide selon la revendication 13, **caractérisée en ce qu'**elle contient au moins une lipase choisie parmi un ou plusieurs polypeptides ayant une séquence d'acides aminés dérivée de la lipase de type sauvage de la souche DSM 4109, dans laquelle au moins l'un des échanges d'acides aminés suivants est présent dans les positions D96L et/ou T213R et/ou N233R, de préférence au moins T213R et N233R.
